# EUROPÄISCHE PATENTSCHRIFT

(11) **EP 4 309 594 B1**
(45) Veröffentlichungstag und Bekanntmachung des Hinweises auf die Patenterteilung: **24.07.2024**
(21) Anmeldenummer: 22186484.6
(22) Anmeldetag: 22.07.2022
(51) Int. Cl.: A61B 17/16, A61B 17/3205, A61B 90/00, A61B 17/29

(54) **KNOCHENSTANZE**
BONE PUNCH
POINÇON POUR OS

(43) Veröffentlichungstag der Anmeldung: 24.01.2024
(73) Patentinhaber: Geister Medizintechnik GmbH, 78532 Tuttlingen (DE)
(72) Erfinder: Geister, Christian, 78532 Tuttlingen (DE)
(74) Vertreter: Stolmár & Partner Patentanwälte PartG mbB

(56) Entgegenhaltungen:
- DE-A1- 10 022 908
- DE-A1- 102010 006 846
- DE-A1- 102011 109 721
- DE-A1- 102013 112 429

## Beschreibung

### Gebiet der Erfindung

Die Erfindung betrifft eine zerlegbare Knochenstanze.

### Stand der Technik

Knochenstanzen sind aus dem Stand der Technik bekannt und werden zur operativen Entfernung von Knochen, Ligamenten, Knorpel und artverwandtem Gewebe in diversen medizinischen Disziplinen eingesetzt.

Die DE 10 2010 06 846 A1 offenbart eine Knochenstanze, an deren proximalem Ende ein festes Griffteil angeordnet ist, und mit einem Schiebeteil, das mittels eines gegen das feste Griffteil verschwenkbar gelagerten schwenkbaren Griffteils axial bewegbar ist, wobei das Schiebeteil innerhalb eines axialen Arbeitshubweges mittels distaler Führungsmittel und proximaler Führungsmittel an dem Schaft in einer Gleitebene anliegend geführt ist, wobei Sperrmittel die axiale Verschiebung des Schiebeteils am proximalen Ende des Arbeitshubweges begrenzen und wobei nach Entriegelung der Sperrmittel das Schiebeteil über das proximale Ende des Arbeitshubweges hinaus in eine Reinigungsstellung bewegbar ist, in welcher die distalen Führungsmittel außer Eingriff kommen, sodass das Schiebeteil von dem Schaft abhebbar ist.

Die DE 100 22 908 A1 offenbart eine Knochenstanze, mit einer in einen ersten Griffteil auslaufenden Lafette und einem an der Lafette geführten, stabförmigen Schieber. Der Schieber ist kraftschlüssig mit dem zweiten Griffteil gekoppelt und mit der Lafette drehbar verbunden. Am ersten Griffteil ist ein Sperrhebel gelagert mit die Griffteile voneinander weg treibenden Federmitteln. Dabei erfolgt die Führung des Schiebers an der Lafette mittels einer in der Nähe des vorderen Lafettenendes angeordneten, im Querschnitt T-förmigen, hinterschnittenen, ersten Längsnut der Lafette und einer im Querschnitt T-förmigen, ersten Rippe des Schiebers. An die erste Längsnut schließt sich griffseitig eine nicht hinterschnittene, zweite Längsnut an, aus der die erste Rippe nach Entriegelung des Sperrhebels heraushebbar ist.

Die DE 10 2013 112 429 A1 offenbart eine Knochenstanze mit zumindest einem Grundkörper, der zumindest eine erste Gleitfläche aufweist, und mit zumindest einem Schiebekörper, der zumindest eine zweite Gleitfläche aufweist, die in zumindest einer Betriebskonfiguration auf der zumindest einen ersten Gleitfläche des zumindest einen Grundkörpers in zumindest eine Verschieberichtung verschiebbar gelagert ist. Es wird vorgeschlagen, dass die medizinische Werkzeugvorrichtung eine Führungseinheit aufweist, die dazu vorgesehen ist, den zumindest einen Schiebekörper zur Herstellung einer Reinigungskonfiguration von der zumindest einen ersten Gleitfläche zumindest teilweise abzuheben. Hierfür ist eine Sperreinheit vorgesehen, die in eine Freigabekonfiguration gebracht wird. In der Betriebskonfiguration wirkt die Sperreinheit als Anschlag zwischen dem Grundkörper und dem Schiebekörper in die Verschieberichtung.

Als hochpreisige Spezialprodukte sind sie für die mehrfache Verwendung ausgelegt und müssen jeweils nach einer Operation gereinigt werden. Um den Reinigungsvorgang zu unterstützen, kann die Knochenstanze zudem zerlegbar ausgebildet sein. Auch zerlegbare Knochenstanzen sind aus dem Stand der Technik bekannt, weisen jedoch im Vergleich zu nicht zerlegbaren Knochenstanzen eine Vielzahl zusätzlicher Komponenten auf, mit denen einerseits eine Zerlegungssicherung, d.h. eine sichere Handhabung während des operativen Gebrauchs ermöglicht werden soll und andererseits deren Zerlegbarkeit. Das Zerlegen selbst und insbesondere das Lösen der Zerlegungssicherung erfolgt dann mehrschrittig. Meist ist es bereits für diesen Schritt notwendig, Komponenten zu entfernen. Ebenfalls muss beim Lösen der Zerlegungssicherung eine bestimmte Reihenfolge der entsprechenden Schritte befolgt werden, da ansonsten die Knochenstanze nicht zerlegbar ist bzw. sogar Schaden nimmt. Außerdem beanspruchen die der Sicherung dienenden Komponenten zusätzlichen Platz, verursachen zusätzliches Gewicht und sind daher beim regulären, d.h. operativen Betrieb der Knochenstanze hinderlich bzw. erschweren diesen.

### Darstellung der Erfindung

Aufgabe der vorliegenden Erfindung ist es daher, eine zerlegbare Knochenstanze bereitzustellen, die eine gute und sichere Handhabbarkeit aufweist sowie robust und einfach zerlegbar ist.

Die Aufgabe wird gelöst durch eine zerlegbare Knochenstanze gemäß Patentanspruch 1. Weitere die Erfindung ausgestaltende Merkmale sind in den abhängigen Patentansprüchen enthalten.

Eine erfindungsgemäße zerlegbare Knochenstanze weist einen Schaft und einen Griff auf, wobei der Schaft ein erstes Schaftteil und ein zweites Schaftteil aufweist, wobei das erste Schaftteil zumindest ein erstes Führungselement aufweist, und das zweite Schaftteil zumindest ein zweites Führungselement aufweist, wobei das erste Schaftteil derart mit dem zweiten Schaftteil verbunden ist, dass das erste Führungselement in Eingriff mit dem zweiten Führungselement ist und das erste Schaftteil parallel zu einer sich von proximal nach distal ergebenden Richtung relativ zum zweiten Schaftteil bewegbar ist, wobei der Schaft in eine Zerlegungsposition, in eine Neutralposition und in eine Stanzposition versetzbar ist, wobei der Griff ein erstes Griffteil und ein zweites Griffteil aufweist, wobei das erste Griffteil bewegbar mit dem zweiten Schaftteil verbunden ist, wobei das zweite Griffteil mit dem zweiten Schaftteil verbunden ist, wobei mittels einer ersten Bewegung des ersten Griffteils, der Schaft von der Neutralposition in die Stanzposition versetzbar ist, dadurch gekennzeichnet, dass mittels einer zweiten Bewegung des ersten Griffteils der Schaft von der Neutralposition in die Zerlegungsposition versetzbar ist.

Derart wird zerlegbare Knochenstanze bereitgestellt, die eine gute und sichere Handhabbarkeit aufweist sowie robust und einfach zerlegbar ist und eine möglichst geringe Anzahl an Bauteilen aufweist.

Die Neutralposition ist die Position, in der sich die Knochenstanze ohne äußere Krafteinwirkung im Betriebszustand befindet.

Die Stanzposition ist die Position, in die die Knochenstanze während deren Hauptverwendung versetzt wird, um das zu entfernende Gewebe zu stanzen. Dafür kann der Schaft ein Stanzelement und eine Stanzaufnahme aufweisen, wobei beim Stanzen, also beim Versetzen des Schaftes in die Stanzposition das Stanzelement in die Stanzaufnahme einfährt, während sich zwischen beiden Elementen das zu entfernende Gewebe befindet. Durch weiteres Einfahren des Stanzelementes in die Stanzaufnahme wird dann das zu entfernende Gewebe entfernt bzw. abgetrennt.

Die Zerlegungsposition ist die Position, in der der Schaft und vorzugsweise die gesamte Knochenstanze in seine/ihre Einzelteile zerlegbar ist. In dieser Position ist insbesondere das erste Schaftteil vom zweiten Schaftteil trennbar und das erste Griffteil vom zweiten Griffteil trennbar.

Die sich von proximal nach distal ergebende bzw. proximal-distale Richtung ist die Richtung, die sich bei der regulären Anwendung der Knochenstanze vom proximalen, d.h. anwendernahen, Ende zum distalen, d.h. anwenderentfernten, Ende ergibt.

Die Knochenstanze ist vorzugsweise derart ausgebildet, dass mit der ersten Bewegung das erste Griffteil auf das zweite Griffteil zu bewegt wird. Vorzugsweise ist die Knochenstanze ferner derart ausgebildet, dass die zweite Bewegung abseits, insbesondere entgegengesetzt zur ersten Bewegung ausführbar ist.

Vorzugsweise ist das erste Griffteil über eine Verbindungsvorrichtung mit dem zweiten Schaftteil verbunden und wird die entsprechende Bewegbarkeit mit Hilfe der Verbindungsvorrichtung ermöglicht.

Vorzugsweise ist das erste Griffteil derart mit dem zweiten Schaftteil verbunden, dass das erste Griffteil um einen sich in der Nähe bzw. im Bereich des zweiten Schaftteils befindenden Punkt rotierbar ist.

Vorzugsweise ist das erste Griffteil ferner derart angeordnet, dass das erste Griffteil, insbesondere dessen Kopfbereich, das erste Schaftteil berührt.

Vorzugsweise ist die Verbindung zwischen dem zweiten Griffteil und dem zweiten Schaftteil im gesamten Betriebszustand fix und auch nicht zur Desinfektion lösbar vorgesehen, d.h. das zweite Griffteil ist nicht relativ zum zweiten Schaftteil bewegbar. Die Verbindung kann eine nicht lösbare Fügeverbindung sein, aber auch eine lösbare Verbindung, wie bspw. eine Steck- oder Schraubverbindung.

Erfindungsgemäß weist das erste Schaftteil einen Verriegelungsbereich auf, wobei der Verriegelungsbereich von einer Neutralstellung in eine Entriegelungsstellung versetzbar ist, wobei der Verriegelungsbereich derart ausgebildet ist, dass, wenn sich der Verriegelungsbereich in der Neutralstellung befindet, ein Versetzen des Schaftes in die Zerlegungsposition verhindert wird und mittels der zweiten Bewegung gleichzeitig der Verriegelungsbereich in die Entriegelungsstellung versetzt wird und der Schaft in die Zerlegungsposition versetzt wird. Derart kann verhindert werden, dass der Schaft ungewollt in die Zerlegungsposition versetzt wird, wodurch die Sicherheit der Knochenstanze erhöht werden kann. Der Verriegelungsbereich kann als Verriegelungsmechanik ausgebildet sein.

Vorzugsweise weist der Verriegelungsbereich einen auslenkbaren Federbereich auf, wobei der Verriegelungsbereich durch Auslenken des Federbereichs in die Entriegelungsstellung versetzbar ist. Derart wird die Zerlegbarkeit der Knochenstanze vereinfacht.

Vorzugsweise ist der Federbereich derart ausgebildet, dass dieser im Wesentlichen senkrecht zu der proximal-distalen Richtung auslenkbar ist. Derart kann verhindert werden, dass im regulären Betrieb, während dem das Schaftteil parallel zu bzw. entlang der proximal-distalen Richtung bewegt wird, ungewollt der Verriegelungsbereich in die Entriegelungsstellung versetzt wird, wodurch wiederum die Sicherheit der Knochenstanze erhöht werden kann.

Vorzugsweise ist der Federbereich im Wesentlichen parallel zu einer horizontalen Richtung auslenkbar. Derart kann in einer vertikalen Richtung eine geringe Höhe der Knochenstanze erreicht werden, wodurch die Handhabbarkeit derselben gesteigert werden kann. Die horizontale Richtung befindet sich in einer horizontalen Ebene und ist senkrecht zu der proximal-distalen Richtung ausgerichtet. Die horizontale Ebene ergibt sich in Bezug auf die Ausrichtung der Knochenstanze im regulären Betrieb bzw. während deren Hauptverwendung. Die horizontale Ebene ist im Wesentlichen parallel zu dem Schaft und senkrecht zu dem Griff ausgerichtet. Die vertikale Richtung ist senkrecht zu der proximal-distalen Richtung und senkrecht zu der horizontalen Richtung ausgerichtet.

In einer bevorzugten Alternative ist der Federbereich im Wesentlichen parallel zu einer vertikalen Richtung auslenkbar. Derart kann in einer horizontalen Richtung eine geringe Breite der Knochenstanze erreicht werden, wodurch die Handhabbarkeit derselben gesteigert werden kann.

Vorzugsweise weist der Federbereich ein loses Ende und ein fixes Ende auf, wobei das lose Ende die größte Auslenkbarkeit aufweist und das fixe Ende nicht auslenkbar ist.

Vorzugsweise weist der Federbereich eine Aussparung auf, die insbesondere abgerundet bzw. gekurvt ausgebildet ist und vorzugsweise am fixen Ende des Federbereiches angeordnet ist. Dadurch kann verhindert werden, dass im Federbereich bei Auslenkung desselben, insbesondere bei regelmäßiger wiederholter Auslenkung, Spannungsrisse resultieren, wodurch die Lebensdauer der Knochenstanze erhöht werden kann. Die Aussparung fungiert dabei als eine Art Freistich.

Vorzugsweise weist das zweite Schaftteil einen Fixierbereich auf, wobei der Fixierbereich derart ausgebildet und mit dem Verriegelungsbereich interagierend angeordnet ist, dass, wenn sich der Verriegelungsbereich in der Neutralstellung befindet, durch den Fixierbereich dem Versetzen des Schaftes in die Zerlegungsposition ein zusätzlicher Widerstand entgegenwirkt. Derart kann zusätzlich verhindert werden, dass der Verriegelungsbereich ungewollt in die Entriegelungsposition versetzt wird, wodurch die Sicherheit der Knochenstanze zusätzlich erhöht werden kann. Als interagierend wird in diesem Zusammenhang insbesondere das entsprechende Zusammenwirken des Fixierbereiches mit dem Verriegelungsbereich verstanden.

Vorzugsweise weist der Verriegelungsbereich zumindest ein erstes Widerstandselement und/oder der Fixierbereich zumindest ein zweites Widerstandselement auf, wobei das erste Widerstandselement und/oder das zweite Widerstandselement derart angeordnet und ausgebildet ist/sind, dass dem Versetzen des Verriegelungsbereiches in die Entriegelungsstellung ein zusätzlicher Widerstand entgegenwirkt. Derart kann die Sicherheit der Knochenstanze zusätzlich erhöht werden.

Vorzugsweise weist die Knochenstanze ein erstes Widerstandselement und ein zweites Widerstandselement auf, wobei das erste Widerstandselement und das zweite Widerstandselement derart interagierend angeordnet und ausgebildet sind, dass dem Versetzen des Verriegelungsbereiches in die Entriegelungsstellung ein zusätzlicher Widerstand entgegenwirkt. Derart kann die Sicherheit der Knochenstanze zusätzlich erhöht werden.

Vorzugsweise ist das erste Widerstandselement als Nase ausgebildet und/oder das zweite Widerstandselement als Wulst ausgebildet. Derart kann die Herstellbarkeit der Knochenstanze vereinfacht werden und deren Robustheit erhöht werden.

In einer bevorzugten Alternative ist das erste Widerstandselement als Wulst ausgebildet und/oder das zweite Widerstandselement als Zylinder ausgebildet. Derart kann die Herstellbarkeit der Knochenstanze vereinfacht werden und deren Robustheit erhöht werden.

Vorzugsweise weist die Knochenstanze ferner ein insbesondere im zweiten Schaftteil angeordnetes erstes Anschlagselement auf, wobei das erste Anschlagselement derart ausgebildet und ferner angeordnet ist, dass die Bewegbarkeit des ersten Griffteils nach proximal hin begrenzt wird. Derart kann verhindert werden, dass der Verriegelungsbereich weiter ausgelenkt bzw. stärker belastet wird, als notwendig bzw. ausgelegt, wodurch wiederum die Standzeit und Sicherheit der Knochenstanze erhöht werden können.

Vorzugsweise weist die Knochenstanze ferner ein insbesondere im zweiten Schaftteil angeordnetes zweites Anschlagselement auf, wobei das zweite Anschlagselement derart ausgebildet und ferner angeordnet ist, dass die Bewegbarkeit des ersten Griffteils nach distal hin begrenzt wird. Derart kann verhindert werden, dass der Schaft bzw. das erste Schaftteil weiter ausgelenkt wird, als die Stanzposition vorgibt bzw. sich aus dieser Stanzposition ergibt, wodurch die Standzeit und die Sicherheit der Knochenstanze erhöht werden können.

Vorzugsweise weist die Knochenstanze ferner ein, insbesondere im zweiten Schaftteil angeordnetes, Rückstellelement auf, wobei das Rückstellelement derart ausgebildet und ferner angeordnet ist, dass nach Ausführen der ersten Bewegung der Schaft automatisch von der Stanzposition wieder in die Neutralposition versetzt wird und insbesondere der ersten Bewegung ein, insbesondere zunehmender, Widerstand entgegenwirkt. Derart muss während der Hauptverwendung der Knochenstanze durch den Bediener zum Zurückstellen bzw. Zurückversetzen des Schaftes von der Stanzposition in die Neutralposition keine Kraft aufgewandt werden, wodurch die Handhabbarkeit gesteigert werden kann. Des Weiteren kann dadurch auf einfache Weise während der Hauptverwendung der Knochenstanze eine Rückmeldung an den Bediener in Bezug auf die momentane Stellung bzw. Positionierung des Schaftes übermittelt werden. Der zunehmende Widerstand kann dadurch erreicht werden, dass das Rückstellelement eine Druckfeder aufweist. Der Widerstand ist dahingehend zunehmend, dass dieser mit Fortschreiten der ersten Bewegung zunimmt, insbesondere linear zunimmt. Der Widerstand beginnt insbesondere unmittelbar nach der Neutralposition bzw. in dieser zuzunehmen. D.h. in der Neutralposition ist der Widerstand am geringsten und unmittelbar vor dem Ende der ersten Bewegung bzw. am Ende der ersten Bewegung, d.h. in der Stanzposition ist der Widerstand am höchsten.

### Kurze Beschreibung der Zeichnungen

Fig. 1a zeigt eine Knochenstanze mit einem proximal angeordneten Verriegelungsbereich in einer Stanzposition.
Fig. 1b zeigt die in Fig. 1a dargestellte Knochenstanze in einer Neutralposition.
Fig. 1c zeigt die in den Fig. 1a und 1b dargestellte Knochenstanze in einer Zerlegungsposition.
Fig. 1d zeigt die in den Fig. 1a bis 1c dargestellte Knochenstanze in einer Aufsicht.
Fig. 1e zeigt die in den Fig. 1a bis 1d dargestellte Knochenstanze in der in Fig. 1b dargestellten Neutralposition in einer Schnittansicht.
Fig. 2a zeigt ein erstes Schaftteil der in den Fig. 1a a bis 1e dargestellten Knochenstanze in einer Seitenansicht.
Fig. 2b zeigt das in Fig. 2a dargestellte erste Schaftteil in einer Ansicht von unten.
Fig. 2c zeigt ein zweites Schaftteil der in den Fig. 1a a bis 1e dargestellten Knochenstanze in einer Seitenansicht.
Fig. 2d zeigt das in Fig. 2c dargestellte zweite Schaftteil in einer Aufsicht.
Fig. 2e zeigt ein erstes Griffteil der in den Fig. 1a a bis 1e dargestellten Knochenstanze.
Fig. 2f zeigt einen Griffkopf des in Fig. 2e gezeigten ersten Griffteils in einer Seitenansicht.
Fig. 2g zeigt das in Fig. 2e dargestellte erste Griffteil in einer Vorderansicht.
Fig. 2h zeigt ein zweites Griffteil der in den Fig. 1a a bis 1e e dargestellten Knochenstanze in einer Seitenansicht.
Fig. 2i zeigt das in Fig. 2h dargestellte zweite Griffteil in einer Ansicht von schräg oben.
Fig. 3a zeigt eine weitere Knochenstanze mit einem distal angeordneten Verriegelungsbereich in einer Stanzposition.
Fig. 3b zeigt die in Fig. 3a dargestellte Knochenstanze in einer Neutralposition.
Fig. 3c zeigt die in den Fig. 3a und 3b dargestellte Knochenstanze in einer Zerlegungsposition.
Fig. 4a zeigt ein erstes Schaftteil der in den Fig. 3a bis 3c dargestellten Knochenstanze in einer Seitenansicht.
Fig. 4b zeigt das in Fig. 4a dargestellte erste Schaftteil von unten.
Fig. 4c zeigt ein zweites Schaftteil der in den Fig. 3a bis 3c dargestellten Knochenstanze in einer Seitenansicht.
Fig. 4d zeigt das in Fig. 4c dargestellte zweite Schaftteil in einer Aufsicht.
Fig. 5a zeigt eine Verbindungsvorrichtung der in den Fig. 1a a bis 1e bzw. 3a bis 3b dargestellten Knochenstanze in einer Explosionsansicht.
Fig. 5b zeigt die in Fig. 5a dargestellte Verbindungsvorrichtung im zusammengebauten Zustand.
Fig. 5c zeigt einen Ausschnitt eines zweiten Schaftteils mit einem ersten Anschlagselement in einer Aufsicht.
Fig. 5d zeigt einen Ausschnitt eines zweiten Schaftteils mit einem zweiten Anschlagselement in einer Aufsicht.
Fig. 5e zeigt ein Rückstellelement in einer Seitenansicht.
Fig. 5f zeigt eine zu dem in Fig. 5e dargestellten Rückstellelement gehörende Feder in einer Seitenansicht.
Fig. 6a zeigt den Schaft der in den Fig. 1a a bis 1e bzw. 3a bis 3c dargestellten Knochenstanze in der in Fig. 1b bzw. 3b dargestellten Neutralposition in einer distalen Detailansicht.
Fig. 6b zeigt das in den Fig. 4a und 4b dargestellte erste Schaftteil in einer Detailansicht.
Fig. 6c zeigt den in Fig. 5d dargestellten Ausschnitt des zweiten Schaftteils mit dem in den Fig. 1a bis 1c und 2a dargestellten Verriegelungsbereich in einer Entriegelungsstellung in einer Aufsicht.
Fig. 6d zeigt einen Ausschnitt des in Fig. 6b dargestellten ersten Schaftteils mit einem Verriegelungsbereich in einer Entriegelungsstellung.
Fig. 6e zeigt eine Alternative zu dem in Fig. 6b dargestellten ersten Schaftteil.

### Beschreibung der bevorzugten Ausführungsformen

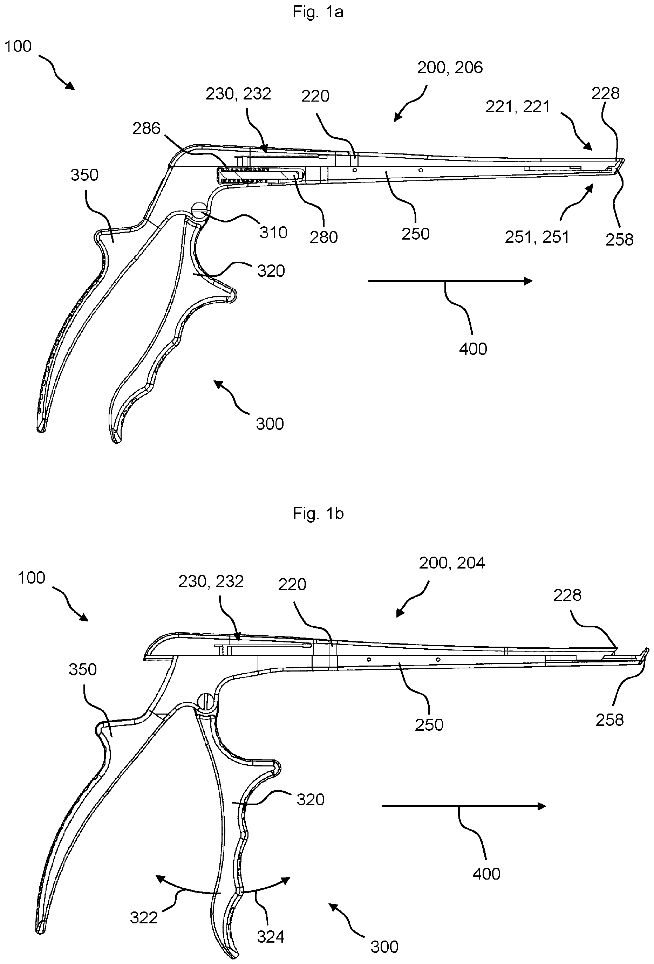

Fig. 1a zeigt eine Knochenstanze 100 mit einem proximal angeordneten Verriegelungsbereich 230 in einer Stanzposition 206. Die Knochenstanze 100 weist einen Griff 300 auf. Der Griff 300 weist ein erstes Griffteil 320 und ein zweites Griffteil 350 auf. Die Knochenstanze 100 weist ferner einen Schaft 200 auf, der sich in der Darstellung in der Stanzposition 206 befindet. Der Schaft 200 weist ein erstes Schaftteil 220 und ein zweites Schaftteil 250 auf. Der Verriegelungsbereich 230 ist Teil des zweiten Schaftteils 250 und befindet sich in einer Neutralstellung 232. Das zweite Schaftteil 250 ist in der Darstellung unterhalb des ersten Schaftteils 220 angeordnet. Das zweite Griffteil 350 ist mit dem zweiten Schaftteil 250 verbunden. Über eine Verbindungsvorrichtung 310 ist das erste Griffteil 320 bewegbar, hier rotierbar, mit dem zweiten Schaftteil 250 verbunden. Die Knochenstanze 100 weist ferner ein Rückstellelement 280 auf, das in dem zweiten Schaftteil 250 angeordnet ist und entlang einer proximal-distalen Richtung 400 ausgerichtet ist. Das Rückstellelement 280 weist ferner eine Feder 286 auf. Im Bereich des Rückstellelements 280 ist in Fig. 1a ein Teilschnitt dargestellt. Die Längsrichtung des Schaftes 200 ist parallel zu der proximal-distalen Richtung 400. Das erste Schaftteil 220 weist ein Stanzelement 228 auf, das am distalen Ende des ersten Schaftteils 220 angeordnet ist. Das zweite Schaftteil 250 weist eine Stanzaufnahme 258 auf, die am distalen Ende des zweiten Schaftteils 250 angeordnet ist. In der dargestellten Stanzposition 206 befindet sich das Stanzelement 228 in der Stanzaufnahme 258 bzw. berührt diese. Das erste Schaftteil 220 weist zwei erste Führungselemente 221 auf, die im distalen Bereich unmittelbar vor dem Stanzelement 228 angeordnet sind. Das zweite Schaftteil 250 weist zwei zweite Führungselemente 251 auf, die im distalen Bereich unmittelbar vor der Stanzaufnahme 258 angeordnet sind.

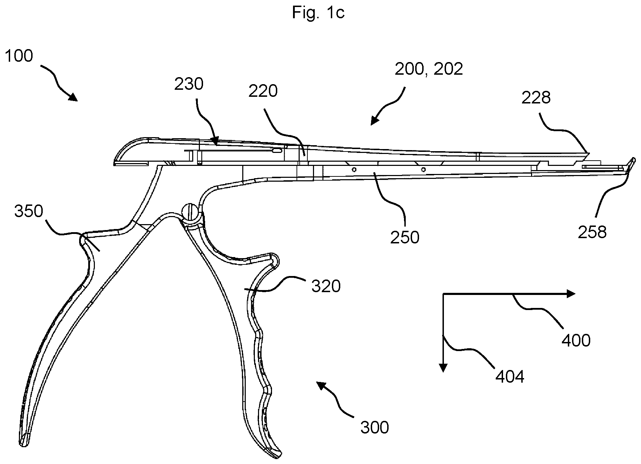

Fig. 1b zeigt die in Fig. 1a dargestellte Knochenstanze 100 in einer Neutralposition 204. In der Neutralposition 204 befindet sich das erste Schaftteil 220 weiter distal, als in der Stanzposition 206 (vgl. Fig. 1a). Dementsprechend ist auch das Stanzelement 228 von der Stanzaufnahme 258 beabstandet, sodass zu stanzendes Gewebe in den distalen Schaftbereich aufnehmbar ist. Wie auch in der Stanzposition 206 befindet sich der Verriegelungsbereich 230 in der Neutralstellung 232. Wird das erste Griffteil 320 im Wesentlichen nach proximal, d.h. entlang einer ersten Bewegung 322 ausgelenkt, wird der Schaft 200 in die Stanzposition 206 bzw. in Richtung dieser ausgelenkt. Die Bewegung 322 folgt einer Kreisbahn, da das erste Griffteil 320 - wie oben im Zusammenhang mit Fig. 1a beschrieben - mit Hilfe der Verbindungsvorrichtung 310 rotierbar mit dem zweiten Schaftteil 250 verbunden ist. Die Rotation erfolgt dabei um eine Achse, die sich im Bereich der Verbindungsvorrichtung 310 befindet. Wird das erste Griffteil 320 nach distal hin ausgelenkt, d.h. eine zweite Bewegung 324 ausgeführt, wird der Schaft 200 in eine Zerlegungsposition 202 versetzt (siehe Fig. 1c). Die zweite Bewegung 324 wird dabei der ersten Bewegung 322 entgegengesetzt ausgeführt und folgt ebenfalls einer Kreisbahn bzw. um die o.g. Achse im Bereich der Verbindungsvorrichtung 310.

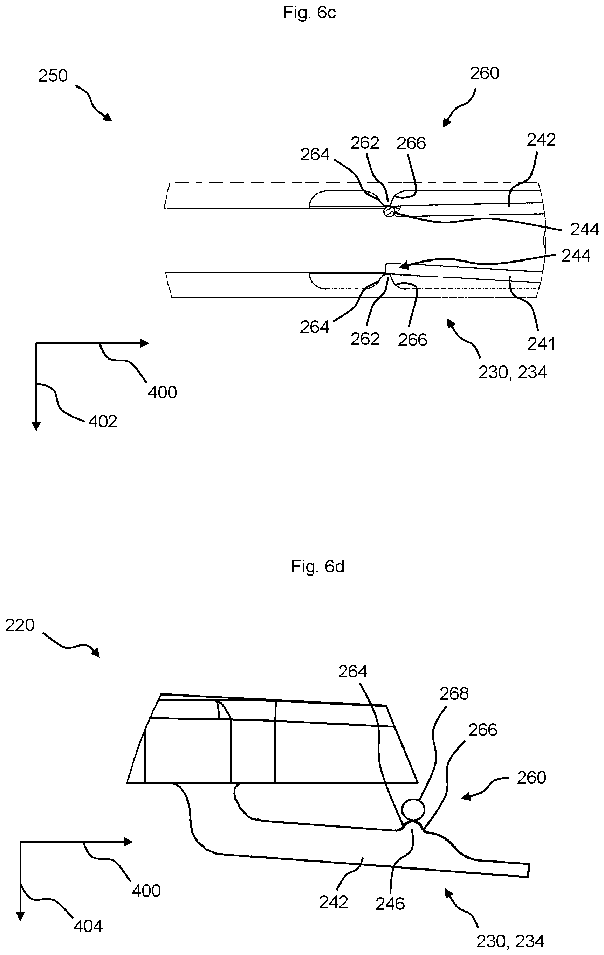

Fig. 1c zeigt die in den Fig. 1a und 1b dargestellte Knochenstanze 100 in der Zerlegungsposition 202. Entlang der proximal-distalen Richtung 400 befindet sich das erste Griffteil 320 maximal von dem zweiten Griffteil 350 entfernt. In der Zerlegungsposition 202 befindet sich das erste Schaftteil 220 entlang einer vertikalen Richtung 404, die senkrecht zu der proximal-distalen Richtung 400 und im Wesentlichen entlang der Längsachse des Griffs 300 ausgerichtet ist, entfernt von dem zweiten Schaftteil 250. Zum Zeitpunkt des Versetzens des Schaftes 200 von der Neutralposition 204 in die Zerlegungsposition 202 wird der Verriegelungsbereich 230 in eine Entriegelungsstellung 234 ausgelenkt (vgl. Fig. 6c).

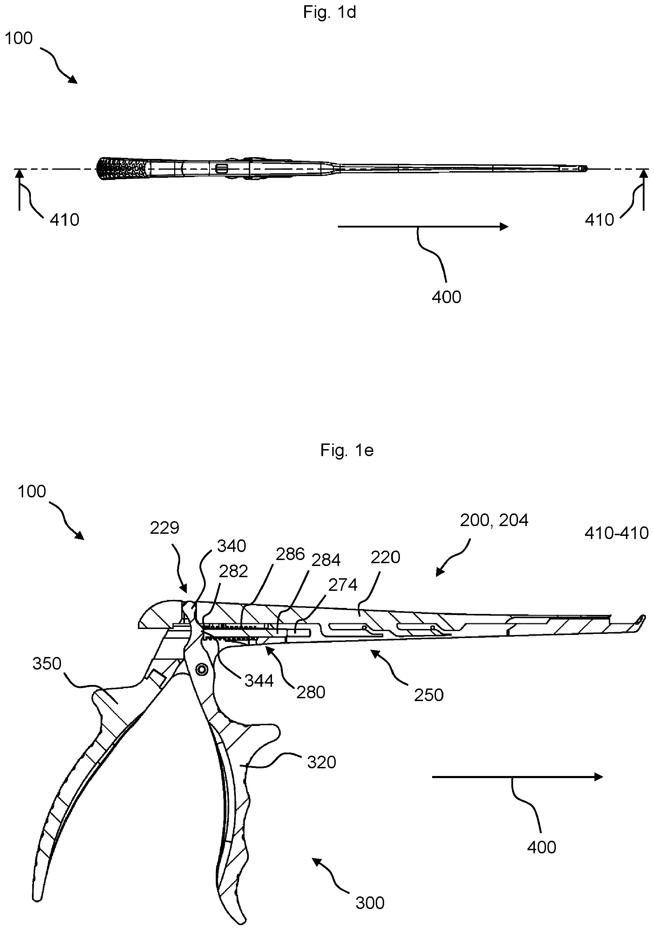

Fig. 1d zeigt die in den Fig. 1a bis 1c dargestellte Knochenstanze 100 in einer Aufsicht. Entlang der proximal-distalen Richtung 400 ist mittig eine Schnittlinie eingezeichnet.

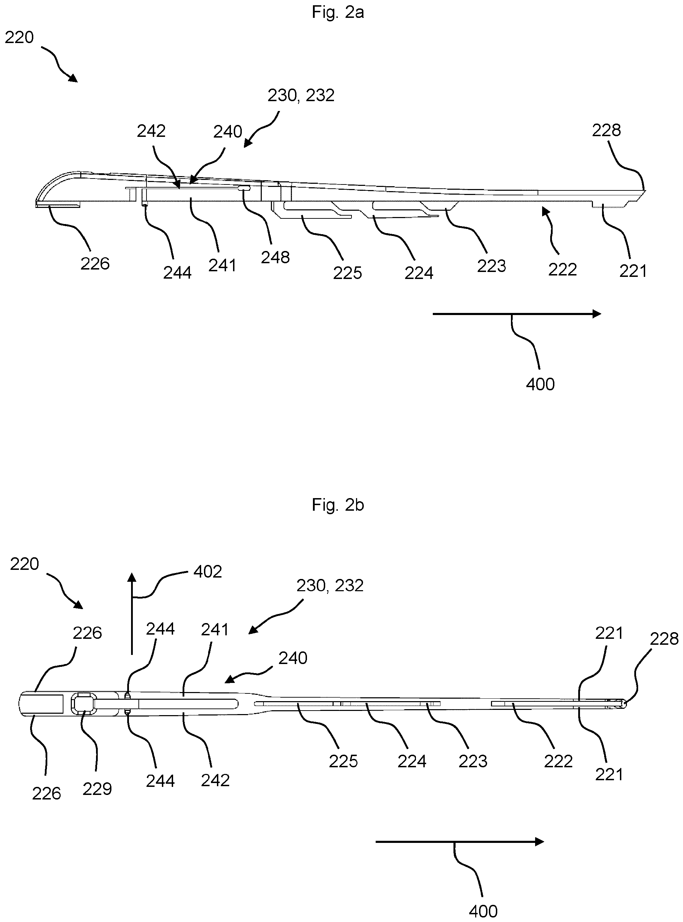

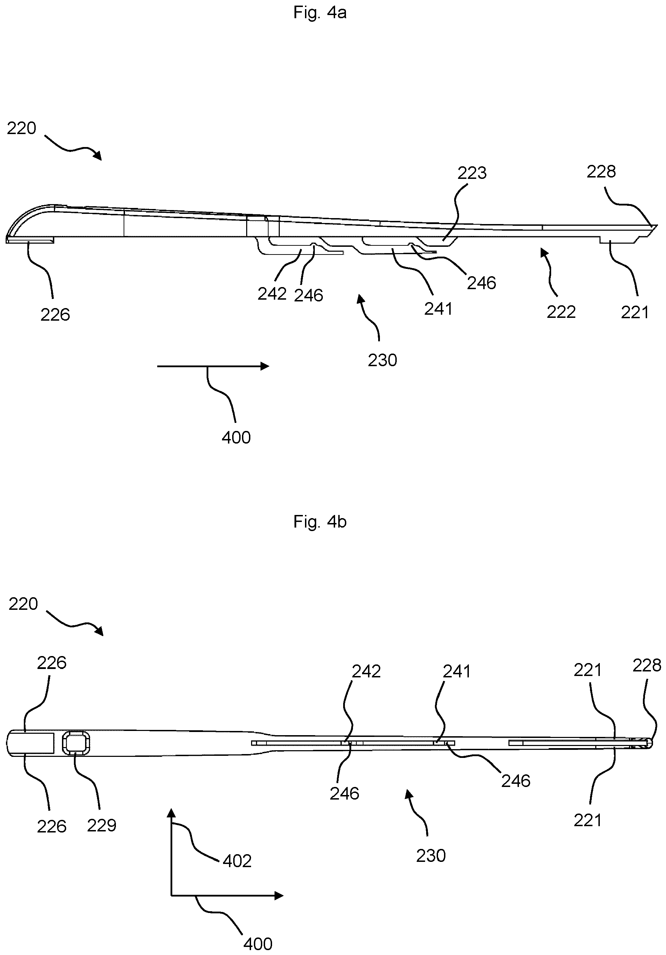

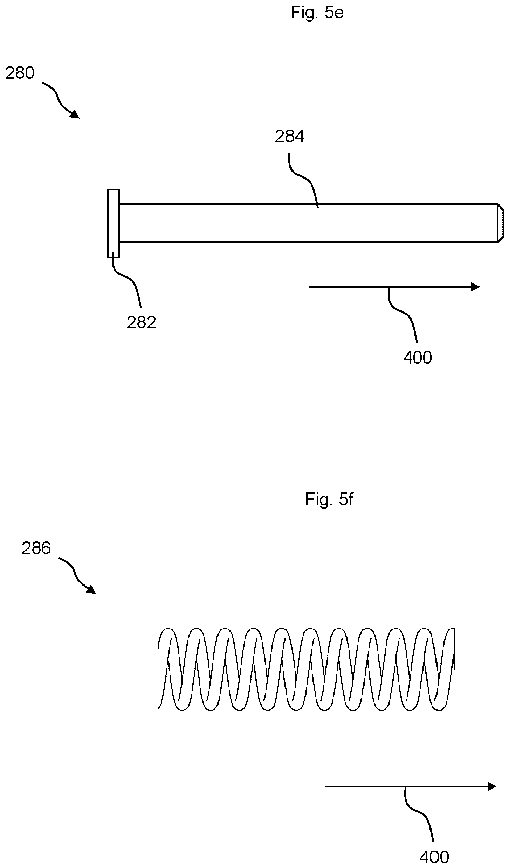

Fig. 1e zeigt die in den Fig. 1a bis 1d dargestellte Knochenstanze 100 in der in Fig. 1b dargestellten Neutralposition 204 in einer Schnittansicht 410-410 entlang der in Fig. 1d dargestellten Schnittlinie. Das Rückstellelement 280 (vgl. Fig. 5e) weist einen Schaft 284 auf der entlang der proximal-distalen Richtung 400 ausgerichtet ist. Das zweite Schaftteil 250 weist ferner eine Führungsöffnung 274 auf, in der das Rückstellelement 280 aufgenommen ist bzw. in der der Schaft 284 geführt wird. Das Rückstellelement 280 weist ferner einen Anschlag 282 auf, der nach proximal hin ausgerichtet ist. Das Rückstellelement 280 weist ferner eine Feder 286 auf, die auf den Schaft 284 aufgesetzt ist. Das erste Griffteil 320 weist im oberen Bereich einen Griffkopf 340 auf. Das erste Schaftteil 220 weist im proximalen Bereich eine Führungsöffnung 229 auf (vgl. Fig. 2b, 4b). Das erste Griffteil 320 weist ferner einen ersten Lastübertragungsbereich 344 auf, der an dem Griffkopf 340 angeordnet ist. Der erste Lastübertragungsbereich 344 ist nach distal hin ausgerichtet und derart berührend mit dem Anschlag 282 angeordnet, dass mittels der ersten Bewegung 322 (vgl. Fig. 1b) das Rückstellelement 280 nach distal hin in die Führungsöffnung 274 bewegt wird. Ferner wird mittels der ersten Bewegung 322 das obere Ende des Griffkopfes 340 nach distal hin bewegt, wodurch über die Führungsöffnung 229 das erste Schaftteil 220 ebenfalls nach distal hin bewegt wird und letztlich der Schaft 200 in die Stanzposition 202 versetzt wird (vgl. Fig. 1a).

Fig. 2a zeigt das erste Schaftteil 220 der in den Fig. 1a bis 1e dargestellten Knochenstanze 100 in einer Seitenansicht. Entgegen der proximal-distalen Richtung 400, d.h. von distal nach proximal weist das erste Schaftteil 220 neben den beiden ersten Führungselementen 221 aufeinander folgend ferner vier weitere erste Führungselemente 222, 223, 224, 225 und zwei weitere Führungselemente 226 auf. Der Verriegelungsbereich 230 befindet sich in der Neutralstellung 232 und weist einen Federbereich 240 auf, der nach distal hin ein fixes, d.h. nicht auslenkbares, Ende aufweist und nach proximal hin ein loses, d.h. maximal auslenkbares Ende aufweist. Der Federbereich 240 weist einen ersten Federarm 241 und einen zweiten Federarm 242 auf, die einander gegenüberliegend angeordnet sind (vgl. Fig. 2b). Der zweite Federarm 242 ist in Fig. 2a vom ersten Federarm 241 verdeckt dargestellt. Der Federbereich 240 weist eine Aussparung 248 auf, die am fixen Ende des Federbereiches 240 angeordnet ist. Der Federbereich 240 ist abgerundet bzw. gekurvt ausgebildet. Der erste Federarm 241 und der zweite Federarm 242 weisen jeweils eine Nase 244 auf, wobei die Nase 244 des zweiten Federarms 242 hier durch die Nase 244 des ersten Federarms 241 verdeckt ist.

Fig. 2b zeigt das in Fig. 2a dargestellte erste Schaftteil 220 in einer Ansicht von unten. Die Führungsöffnung 229 ist von unten nach oben durchgängig ausgebildet. Der erste Federarm 241 und der zweite Federarm 242 sind jeweils so ausgebildet, dass deren lose Enden, d.h. deren proximale Enden entlang einer horizontalen Richtung 402 auslenkbar sind. Der Verriegelungsbereich 230 befindet sich in der Neutralstellung 232.

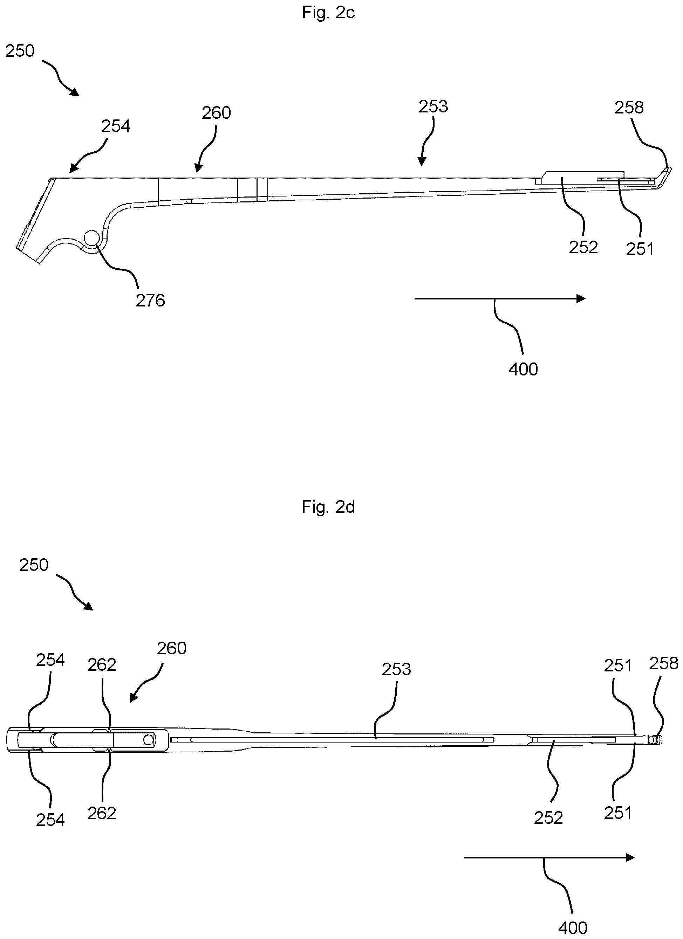

Fig. 2c zeigt das zweite Schaftteil 250 der in den Fig. 1a a bis 1e dargestellten Knochenstanze 100 in einer Seitenansicht. Entgegen der proximal-distalen Richtung 400, d.h. von distal nach proximal weist das zweite Schaftteil 250 neben den beiden zweiten Führungselementen 251 aufeinander folgend ferner ein weiteres zweites Führungselement 252, ein weiteres zweites Führungselement 253 und zwei weitere Führungselemente 254 auf. Die ersten Führungselemente 221, 222, 223, 224, 225, 226 und die zweiten Führungselemente 251, 252, 253, 254 sind zueinander derart komplementär ausgebildet, dass das erste Schaftteil 220 entlang der proximal-distalen Richtung 400 entlang des zweiten Schaftteils 250 bewegbar ist, d.h. von der Neutralposition 204 in die Stanzposition 206 versetzbar ist und umgekehrt. Die beiden ersten Führungselemente 221 und die beiden zweiten Führungselement 251 befinden sich in der Stanzposition 202 und in der Neutralposition 204 miteinander in Eingriff, ebenso wie das erste Führungselement 222 und das zweite Führungselement 252, die ersten Führungselemente 223, 224, 225 und das zweite Führungselement 253, und die beiden ersten Führungselemente 226 und die beiden zweiten Führungselemente 254. Das zweite Schaftteil 250 weist ferner einen Fixierbereich 260 auf, der komplementär zu dem Federbereich 240 ausgebildet ist (vgl. Fig. 2a, 2b). Das zweite Schaftteil 250 weist ferner eine Aufnahme 276 auf, die zur Aufnahme der Verbindungsvorrichtung 310 ausgebildet ist (vgl. Fig. 1a).

Fig. 2d zeigt das in Fig. 2c dargestellte zweite Schaftteil 250 in einer Aufsicht. Der Fixierbereich 260 weist zwei gegenüberliegende Wülste 262 auf.

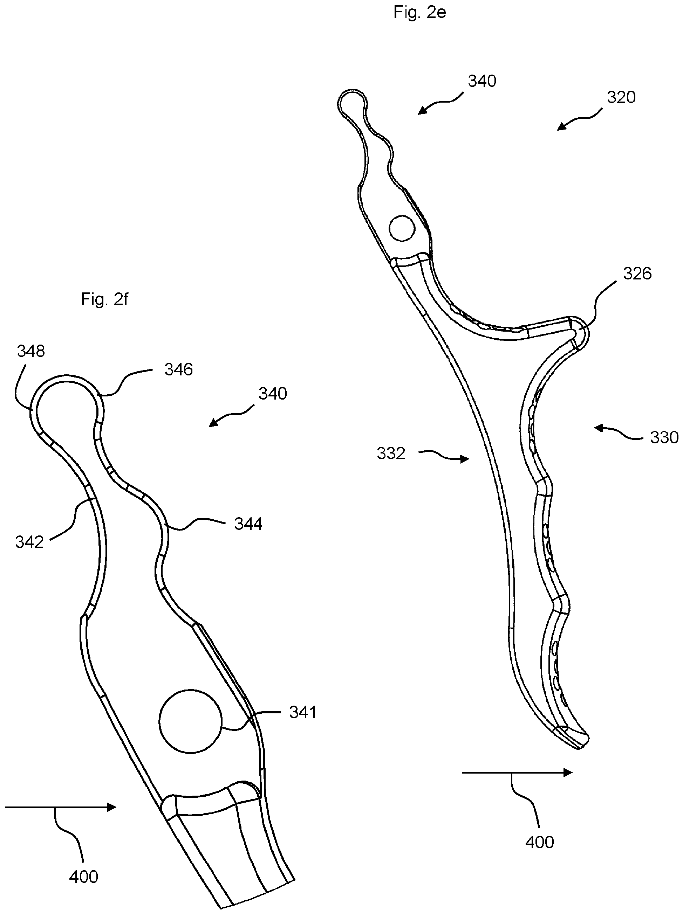

Fig. 2e zeigt das erste Griffteil 320 der in den Fig. 1a a bis 1e dargestellten Knochenstanze 100 entsprechend der bei der Zerlegungsposition 202 vorherrschenden Ausrichtung. Das erste Griffteil 320 weist eine erste Griffseite 330 auf, die nach distal hin orientiert ist und eine zweite Griffseite 332 auf, die nach proximal hin orientiert ist. Auf der ersten Griffseite 330 weist das erste Griffteil 320 einen Vorsprung 326 auf, der während der Hauptverwendung der Knochenstanze 100 dem Benutzer einen sichereren und ergonomischen Halt ermöglicht. Oberhalb des Vorsprungs 326 ist die Positionierung des Zeigefingers des Benutzers vorgesehen und unterhalb des Vorsprungs die Positionierung des Mittel-, Ring- und kleinen Fingers des Benutzers.

Fig. 2f zeigt den Griffkopf 340 des in Fig. 2e gezeigten ersten Griffteils 320 in einer Seitenansicht. Der Griffkopf 340 weist eine Aufnahme 341 auf, die zur Aufnahme der Verbindungsvorrichtung 310 ausgebildet ist (vgl. Fig. 1a) und mit der Aufnahme 276 des zweiten Schaftteils 250 in Deckung gebracht wird (vgl. Fig. 2c). Im oberen Bereich weist der Griffkopf 340 ferner einen zweiten Lastübertragungsbereich 346 und einen dritten Lastübertragungsbereich 348 auf. Der zweite Lastübertragungsbereich 346 ist nach distal hin ausgerichtet und ist derart ausgebildet, dass während der ersten Bewegung 322 (vgl. Fig. 1b) auf die distale Wand der Führungsöffnung 229 Last übertragbar ist (vgl. Fig. 2b), wodurch wiederum das erste Schaftteil 220 nach distal hin bewegt wird. Der dritte Lastübertragungsbereich 348 ist nach proximal hin ausgerichtet und derart ausgebildet, dass auf die proximale Wand der Führungsöffnung 229 Last übertragbar ist (vgl. Fig. 2b), wodurch wiederum das erste Schaftteil 220 nach proximal hin bewegbar ist und von der Neutralposition 204 in die Zerlegungsposition 202 versetzbar ist. Der Kopfbereich 340 weist ferner eine Aussparung 340 auf, mit der bewegte Massen reduziert werden, wodurch die Handhabbarkeit der Knochenstanze 100 verbessert werden kann. Der erste Lastübertragungsbereich 344, der zweite Lastübertragungsbereich 346 und der dritte Lastübertragungsbereich 348 sind jeweils gekrümmt bzw. gekurvt ausgebildet. Dadurch kann erreicht werden, dass bei der Übertragung von Last ausgehend vom ersten Griffteil 320 entlang einer Kreisbahn (vgl. Fig. 1b) auf das erste Schaftteil 220 und auf das Rückstellelement 280, bei denen eine im Wesentlichen lineare Bewegung erzeugt wird, kein Verkanten auftritt bzw. der Griffkopf 340 am ersten Schaftteil 220 und am Rückstellelement 280 abrollt. Dadurch kann die Robustheit der Knochenstanze 100 gesteigert und deren Handhabbarkeit verbessert werden.

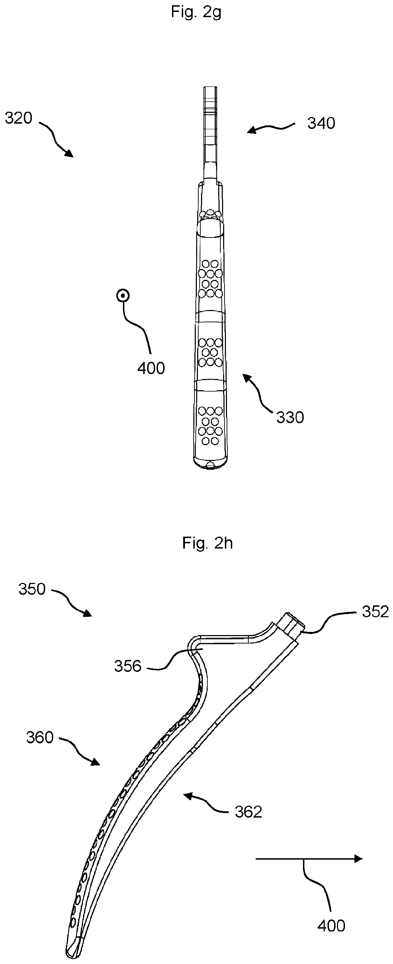

Fig. 2g zeigt das in Fig. 2e dargestellte erste Griffteil 320 in einer Vorderansicht, d.h. entgegen der proximal-distalen Richtung 400. Die erste Griffseite 330 ist breiter als der Griffkopf 340. Der Griffkopf 340 ist derart schmal ausgebildet, dass er in das zweite Schaftteil 250 (vgl. Fig. 2c, 2d) aufnehmbar ist.

Fig. 2h zeigt das zweite Griffteil 350 der in den Fig. 1a a bis 1e dargestellten Knochenstanze 100 in einer Seitenansicht. Das zweite Griffteil 350 weist eine erste Griffseite 360 auf, die nach proximal hin orientiert ist und eine zweite Griffseite 362 auf, die nach distal hin orientiert ist. Auf der ersten Griffseite 360 weist das zweite Griffteil 350 einen Vorsprung 356 auf, der während der Hauptverwendung der Knochenstanze 100 dem Benutzer einen sichereren und ergonomischen Halt ermöglicht. Der Vorsprung 356 ist dazu ausgebildet, ein Nachobenrutschen der Hand des Benutzers hin zu dem proximalen Ende des Schaftes 200 zu verhindern. Dazu ist vorgesehen, dass der Mittelhandbereich des Benutzers zwischen Daumen und Zeigefinger unmittelbar unterhalb des Vorsprungs 356 anliegt. Das zweite Griffteil 350 weist ferner ein Steckelement 352 auf, das im oberen Bereich des zweiten Griffteils 350 angeordnet ist und mit Hilfe dessen das zweite Griffteil 350 mit dem zweiten Schaftteil 250 verbunden bzw. gefügt wird (vgl. Fig. 1a).

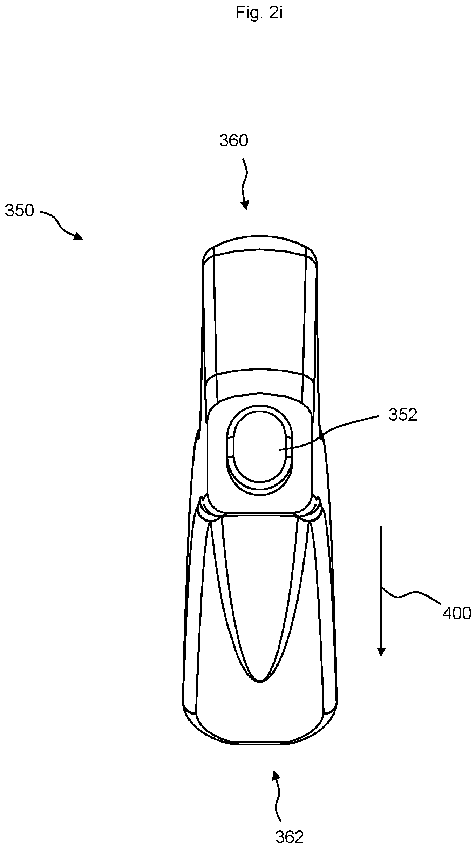

Fig. 2i zeigt das in Fig. 2h dargestellte zweite Griffteil 350 in einer Ansicht von schräg oben. Die erste Griffseite 360 ist in der Darstellung nach oben ausgerichtet und die zweite Griffseite 362 nach unten. Das Steckelement 352 weist einen ovalen Querschnitt auf. Derart kann eine gute Zusammensetzbarkeit des zweiten Griffteils 350 und des zweiten Schaftteils 250 erreicht werden. Es ist ebenfalls möglich, dass das Steckelement 352 andere Querschnitte aufweist, die bspw. die Lastübertragung zwischen dem zweiten Schaftteil 250 und dem zweiten Griffteil unterstützen bzw. verbessern oder eine Verformung zwischen diesen beiden Komponenten vermindern. Solche Querschnitte können beispielsweise runde, polygonale aber auch kreuz- oder sternförmige Querschnitte sein.

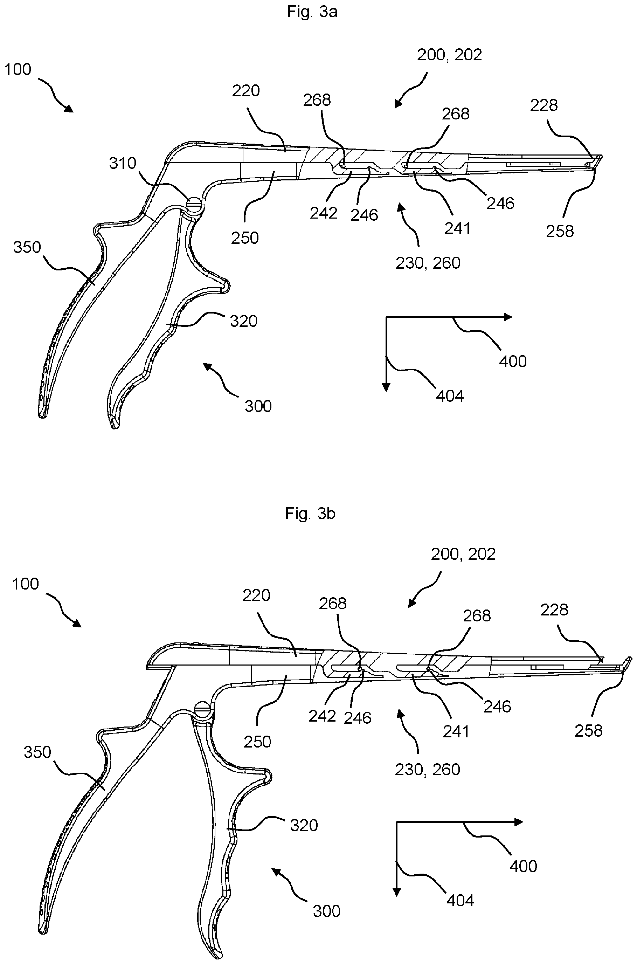

Fig. 3a zeigt eine weitere Knochenstanze 100 mit einem distal angeordneten Verriegelungsbereich 230 in einer Stanzposition 202. Im Bereich des Verriegelungsbereiches 230 ist die Knochenstanze 100 im Teilschnitt dargestellt. Der erste Federarm 241 und der zweite Federarm 242 sind entlang der proximal-distalen Richtung 400 hintereinander angeordnet, wobei der erste Federarm 241 weiter distal angeordnet ist als der zweite Federarm 242. Der Verriegelungsbereich 230 und der Fixierbereich 260 sind miteinander in Eingriff stehend angeordnet. Der Fixierbereich 260 weist zwei Zylinder 268 auf. Der erste Federarm 241 und der zweite Federarm 242 weisen jeweils eine Wulst 246 auf. Die beiden Zylinder 268 befinden sich jeweils am proximalen Ende des ersten und zweiten Federarms 241, 242 bzw. an deren fixen Enden.

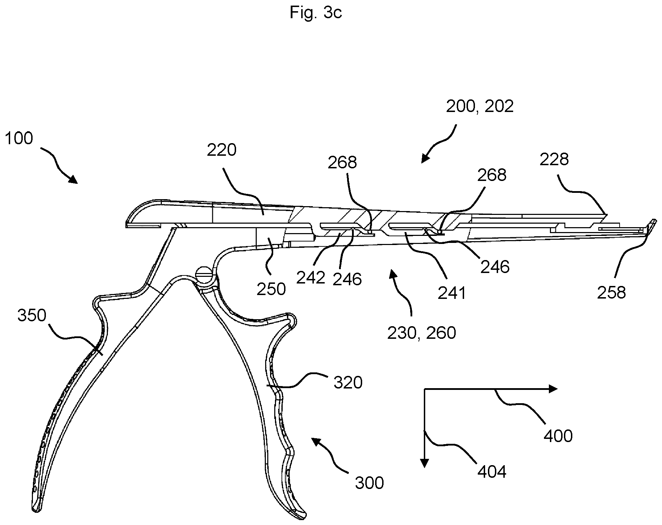

Fig. 3b zeigt die in Fig. 3a dargestellte Knochenstanze 100 in einer Neutralposition 204. Im Bereich des Verriegelungsbereiches 230 ist die Knochenstanze 100 im Teilschnitt dargestellt. Die beiden Zylinder 268 befinden sich jeweils unmittelbar vor den beiden Wülsten 246, d.h. weiter proximal als die beiden Wülste 246. Dementsprechend müssten vor einem weiteren Versatz des ersten Schaftteils 220 nach proximal hin die beiden Zylinder 268 die beiden Wülste 246 überwinden. Dazu müssten der erste und der zweite Federarm 241, 242 entlang der vertikalen Richtung 404 ausgelenkt werden, wodurch wiederum Platz für die beiden Zylinder 268 geschaffen würde, um an den beiden Wülsten 246 vorbeizugleiten. Dieser Zustand ist in Fig. 3c dargestellt.

Fig. 3c zeigt die in den Fig. 3a und 3b dargestellte Knochenstanze in einer Zerlegungsposition 202. Im Bereich des Verriegelungsbereiches 230 ist die Knochenstanze 100 im Teilschnitt dargestellt. Die beiden Zylinder 268 befinden sich jeweils distal von den jeweils zu ihnen gehörenden Wülsten 246. Das erste Schaftteil 220 befindet sich vertikal von dem zweiten Schaftteil 250 entfernt, was dadurch erreicht wird, dass der erste und zweite Federarm 241, 242 jeweils distal entlang der vertikalen Richtung 404 verjüngend ausgebildet sind und der jeweils entsprechend oberhalb des ersten und zweiten Federarms 241, 242 befindliche Teil des ersten Schaftteils 220 in gleichem Maße verdickend ausgebildet ist.

Fig. 4a zeigt ein erstes Schaftteil 220 der in den Fig. 3a bis 3c dargestellten Knochenstanze 100 in einer Seitenansicht. Das in Fig. 4a dargestellte erste Schaftteil 220 ist ähnlich zu dem in den Fig. 2a und 2b dargestellten ersten Schaftteil 220 ausgebildet, wobei das in Fig. 4a dargestellte erste Schaftteil 220 lediglich die ersten Führungselemente 221, 222, 223, 226 aufweist. Anstelle der bei dem in den Fig. 2a und 2b dargestellten ersten Schaftteil 220 vorhandenen ersten Führungselemente 224, 225 sind bei dem in Fig. 4a dargestellten ersten Schaftteil 220 der erste und der zweite Federarm 241, 242 vorgesehen bzw. der Verriegelungsbereich 230 vorgesehen.

Fig. 4b zeigt das in Fig. 4a dargestellte erste Schaftteil 220 von unten. Der erste und der zweite Federarm 241, 242 sind in Bezug auf die horizontale Richtung 402 zentriert im ersten Schaftteil 220 angeordnet. Der erste und zweite Federarm 241, 242 sind ferner derart ausgebildet, dass sie - wie auch die ersten Führungselemente 224, 225 des in den Fig. 2a und 2b dargestellten ersten Schaftteils 220 - eine Führung des ersten Schaftteils 220 entlang der proximal-distalen Richtung 400 begünstigen.

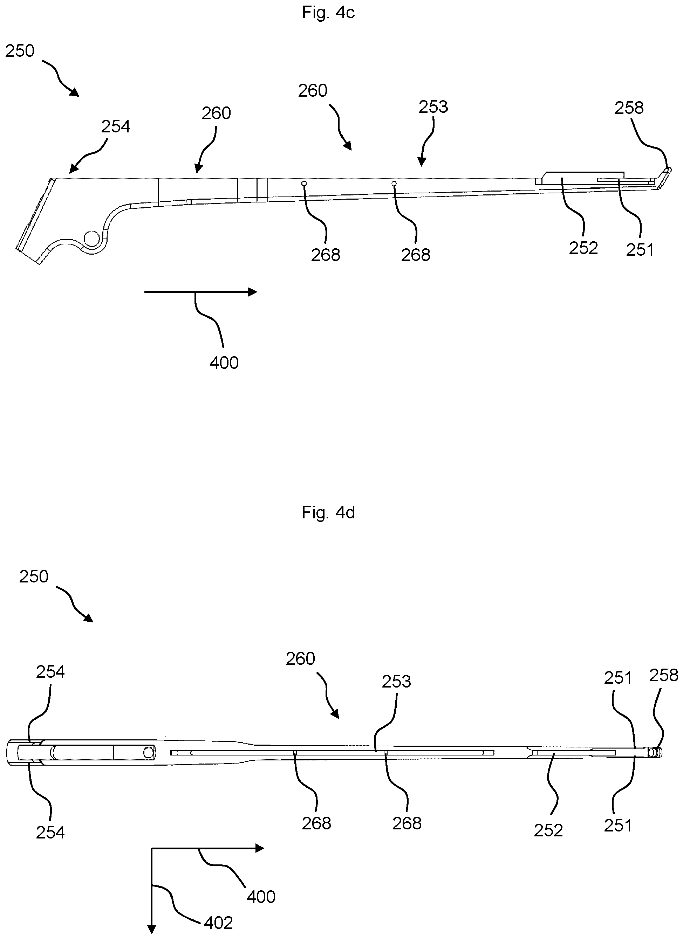

Fig. 4c zeigt ein zweites Schaftteil 250 der in den Fig. 3a bis 3c dargestellten Knochenstanze 100 in einer Seitenansicht. Im Vergleich zu dem in den Fig. 2c und 2d dargestellten zweiten Schaftteil 250 weist das in Fig. 4c dargestellte zweite Schaftteil 250 zusätzlich die beiden Zylinder 268 auf, die in dem zweiten Führungselement 253 angeordnet sind.

Fig. 4d zeigt das in Fig. 4c dargestellte zweite Schaftteil 250 in einer Aufsicht. Die beiden Zylinder 268 sind derart ausgerichtet, dass deren Längsachsen entlang der horizontalen Richtung 402, d.h. vertikal zur proximal-distalen Richtung 400 verlaufen.

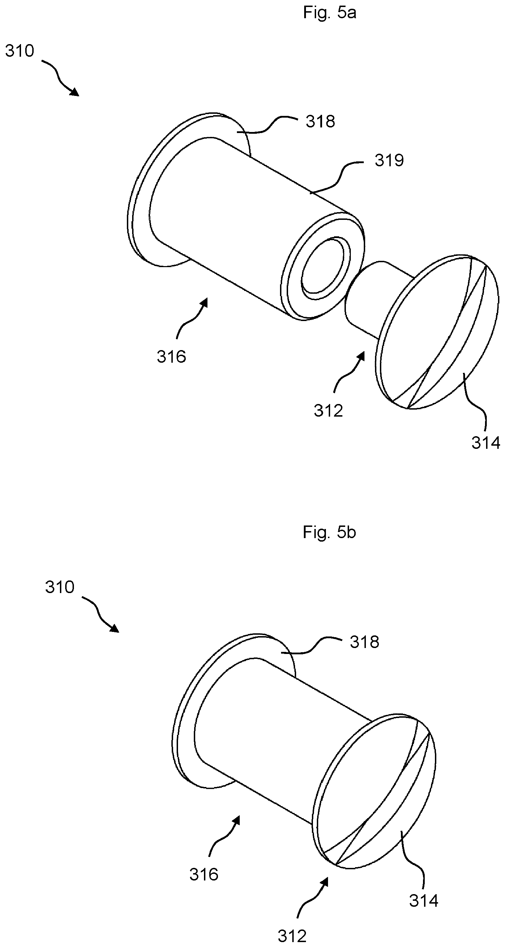

Fig. 5a zeigt eine Verbindungsvorrichtung 310 der in den Fig. 1a bis 1e bzw. 3a bis 3b dargestellten Knochenstanze 100 in einer Explosionsansicht. Die Verbindungsvorrichtung 310 weist einen Verbindungseinsatz 312 und ein Gegenstück 316 auf. Der Verbindungseinsatz 312 weist einen Anschlag 314 auf. Das Gegenstück 316 weist ebenfalls einen Anschlag 318 auf. Das Gegenstück 316 weist ferner eine Aufnahme 319 auf, in die der Verbindungseinsatz 312 einführbar ist. Der Verbindungseinsatz 312 ist dabei so weit einführbar, bis dessen Anschlag 314 an das dem Anschlag 318 des Gegenstücks 316 gegenüberliegende Ende der Aufnahme 319 anliegt bzw. anstößt. Bei der Verbindungsvorrichtung 310 kann dabei insbesondere eine Schraubvorrichtung vorgesehen sein, d.h. der Verbindungseinsatz 312 weist ferner ein Außengewinde auf und die Aufnahme 319 ein dazu passendes Innengewinde. Derart kann eine variable und einfach handhabbare Verbindungsvorrichtung bereitgestellt werden. Bei der Verbindungsvorrichtung 310 kann aber auch eine Pressverbindung vorgesehen sein, d.h. die Aufnahme 319 weist einen geringeren Innendurchmesser auf, als der Verbindungseinsatz 312. Des Weiteren kann bei der Verbindungsvorrichtung 310 auch eine Schweiß- oder eine Nietverbindung vorgesehen sein.

Fig. 5b zeigt die in Fig. 5a dargestellte Verbindungsvorrichtung 310 im zusammengebauten Zustand. Die Verbindungsvorrichtung 310 ist dazu ausgebildet, dass die Anschläge 314, 318 im zusammengebauten Zustand spielfrei bzw. nahezu spielfrei jeweils außen an dem zweiten Schaftteil 250 anliegen. Die Aufnahme 319 ist dazu ausgebildet, das zweite Schaftteil 250 und das erste Griffteil 320 zu lagern. Dazu ist die Außenkontur der Aufnahme 319 derart komplementär zur Innenkontur der Aufnahme 276 des zweiten Schaftteils 250 (vgl. Fig. 2c) und zur Innenkontur der Aufnahme 341 des ersten Griffteils 320 (vgl. Fig. 2f) ausgebildet, dass einerseits das erste Griffteil 320 sicher im zweiten Schaftteil 250 gelagert ist und andererseits das erste Griffteil 320 mit geringem Widerstand und geringem Spiel bzw. spielfrei in dem zweiten Schaftteil 250 rotierbar ist.

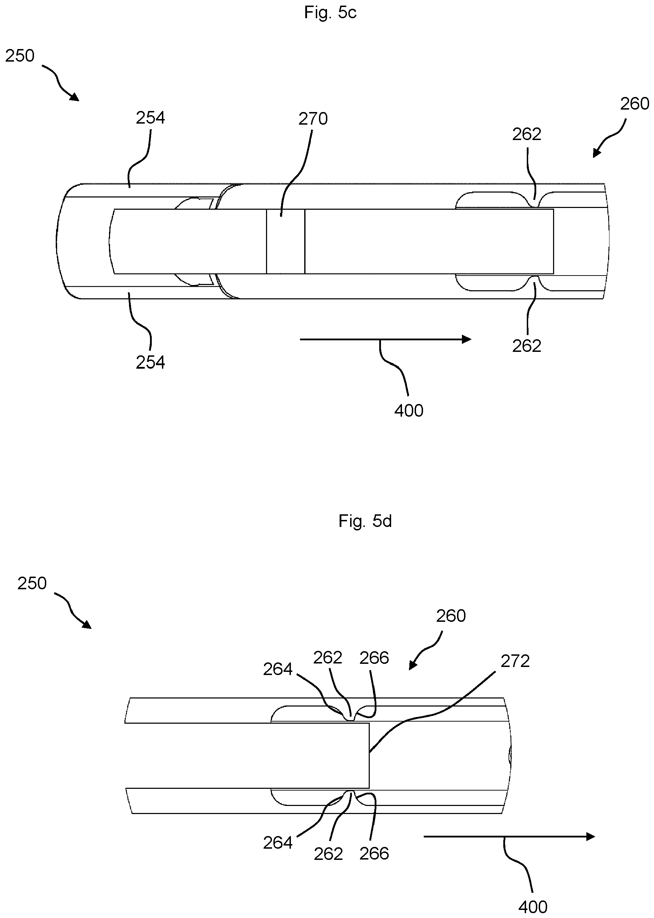

Fig. 5c zeigt einen Ausschnitt des zweiten Schaftteils 250 mit einem ersten Anschlagselement 270 in einer Aufsicht. Die Darstellung zeigt zwar ein Detail des in den Fig. 2c und 2d dargestellten zweiten Schaftteils 250, ist jedoch ebenso auf das in den Fig. 4c und 4d dargestellte zweiten Schaftteils 250 übertragbar. Das erste Anschlagselement 270 ist dazu ausgebildet und derart angeordnet, dass die Bewegbarkeit des ersten Griffteils 320, insbesondere des Griffkopfes 340, im Zuge der zweiten Bewegung 324 nach proximal hin beschränkt wird. Dadurch kann eine Beschädigung bzw. ein Verklemmen der gegeneinander bewegten Komponenten vermieden werden, insbesondere des ersten Griffteils 320 und des ersten Schaftteils 220.

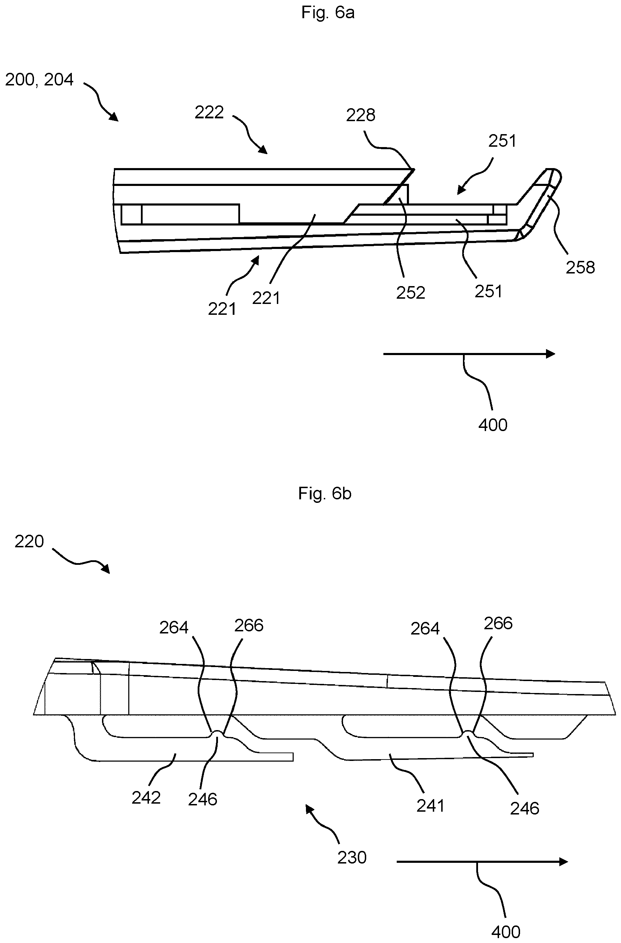

Fig. 5d zeigt einen Ausschnitt des zweiten Schaftteils 250 mit einem zweiten Anschlagselement 272 in einer Aufsicht. Die Darstellung zeigt zwar ein Detail des in den Fig. 2c und 2d dargestellten zweiten Schaftteils 250, ist jedoch ebenso auf das in den Fig. 4c und 4d dargestellte zweiten Schaftteils 250 übertragbar. Das zweite Anschlagselement 272 ist dazu ausgebildet und derart angeordnet, dass die Bewegbarkeit des ersten Griffteils 320, insbesondere des Griffkopfes 340, im Zuge der ersten Bewegung 322 nach distal hin beschränkt wird. Dadurch kann eine Beschädigung bzw. ein Verklemmen der gegeneinander bewegten Komponenten vermieden werden, insbesondere des ersten Griffteils 320 und des ersten Schaftteils 220. Fig. 5d zeigt ferner Details des Fixierbereiches 260 bzw. der Wülste 262. Die beiden Wülste 262 weisen jeweils eine proximale Flanke 264 und eine distale Flanke 266 auf. Die beiden distalen Flanken 266 sind dabei derart ausgebildet, dass in der Neutralposition 204 (vgl. Fig. 1b), wenn im Zuge der zweiten Bewegung 324 die Nasen 244 in dem Fixierbereich 260 von distal nach proximal geführt werden, die Nasen 244 an den distalen Flanken 266 anliegen und vorerst ein weiterer Versatz des ersten Schaftteils 220 nach proximal hin verhindert wird. Wird dann in ausreichendem Maße Kraft aufgebracht und die zweite Bewegung 324 fortgesetzt, fahren die Nasen 244 an den distalen Flanken 266 entlang, wodurch der erste und der zweite Federarm 241, 242 jeweils entlang bzw. entgegen der horizontalen Richtung 402 ausgelenkt werden. Derart kann die Entriegelungsstellung 234 erreicht werden, d.h. können die Nasen 244 die distalen Flanken 266 überwinden und an den proximalen Flanken 264 nach proximal hin abgleiten, wodurch der Schaft 200 in die Zerlegungsposition 202 versetzt wird (vgl. Fig. 1c). Soll der Schaft 200 anschließend wieder entgegen der zweiten Bewegung 324 in die Neutralposition 204 versetzt werden, muss ausreichend Kraft aufgebracht werden, damit die Nasen 244 die proximalen Flanken 264 überwinden können. Die beiden proximalen Flanken 264 weisen dabei entlang der proximal-distalen Richtung 400 einen geringeren Anstieg als die beiden distalen Flanken 266 auf. Derart wird es ermöglicht, dass dem Versetzen des Schaftes 200 von der Neutralposition 204 in die Zerlegungsposition 202 ein größerer Widerstand entgegengebracht wird, als dem Versetzen des Schaftes 200 von der Zerlegungsposition 202 in die Neutralposition 204. Derartig ausgebildete proximale und distale Flanken 264, 266 können ebenfalls bei dem in den Fig. 4a und 4b dargestellten ersten Schaftteil 220 vorgesehen sein (vgl. Fig. 6b).

Fig. 5e zeigt ein Rückstellelement 280 in einer Seitenansicht. Der Anschlag 282 ist ferner dazu ausgebildet, ein Eindringen des Rückstellelementes 280 in die Führungsöffnung 274 nach distal hin zu begrenzen (vgl. Fig. 1e).

Fig. 5f zeigt die zu dem in Fig. 5e dargestellten Rückstellelement 280 gehörende Feder 286 in einer Seitenansicht. Die Feder 286 wird dabei auf den Schaft 284 aufgesetzt (vgl. Fig. 1e) und ist dazu ausgebildet, der ersten Bewegung 322 einen Widerstand, insbesondere einen zunehmenden Widerstand entgegenzusetzen. Des Weiteren kann mit der Feder 286 erreicht werden, dass
während der Hauptverwendung der Knochenstanze 100 durch den Bediener zum Zurückstellen bzw. Zurückversetzen des Schaftes 200 von der Stanzposition 206 in die Neutralposition 204 keine Kraft aufgewandt werden, wodurch die Handhabbarkeit gesteigert werden kann.

Fig. 6a zeigt den Schaft 200 der in den Fig. 1a bis 1e bzw. 3a bis 3c dargestellten Knochenstanze 100 in der in Fig. 1b bzw. 3b dargestellten Neutralposition 204 in einer distalen Detailansicht. Mittels des Zusammenwirkens der beiden am distalen Ende angeordneten ersten Führungselemente 221 mit den beiden am distalen Ende angeordneten zweiten Führungselementen 251 wird eine sichere Führung des ersten Schaftteils 220 im äußeren Bereich ermöglicht und mittels des Zusammenwirkens des etwas weiter proximal angeordneten ersten Führungselementes 222 und zweiten Führungselementes 252 eine sichere Führung im mittleren Bereich (vgl. Fig. 2a bis 2d). Derart wird ein präzises und insbesondere unter Belastung sicheres Aufeinanderzubewegen des Stanzelementes 228 und der Stanzaufnahme 258 ermöglicht.

Fig. 6b zeigt das in den Fig. 4a und 4b dargestellte erste Schaftteil 220 in einer Detailansicht. Die beiden Wülste 246 weisen jeweils eine proximale Flanke 264 und eine distale Flanke 266 auf, wobei in dem in Fig. 6b dargestellten ersten Schaftteil 220 zum Versetzen des Schaftes 200 in die Zerlegungsposition 202 mit den Zylindern 268 die beiden proximalen Flanken 264 überwunden werden, nicht aber die beiden distalen Flanken 266, wie bei dem in den Fig. 5d sowie 2c und 2d dargestellten zweiten Schaftteil 250.

Fig. 6c zeigt den in Fig. 5d dargestellten Ausschnitt des zweiten Schaftteils 250 mit dem in den Fig. 1a bis 1c und 2a dargestellten Verriegelungsbereich 230 in der Entriegelungsstellung 234 in einer Aufsicht. Der erste Federarm 241 und der zweite Federarm 242 sind dabei derart ausgerichtet, dass sich die beiden Nasen 244 jeweils auf der Spitze der beiden Wülste 262 befinden, d.h. zwischen der proximalen Flanke 264 und der distalen Flanke 266. Dadurch wird der erste Federarm 241 entgegen der horizontalen Richtung 402, d.h. nach innen, und der zweite Federarm 242 mit der horizontalen Richtung 402, d.h. ebenfalls nach innen, ausgelenkt, wodurch das erste Schaftteil 220 weiter nach proximal, d.h. entgegen der proximal-distalen Richtung 400 ausgelenkt bzw. versetzt werden kann. Dadurch kann der Schaft 200 von der Neutralposition 204 in die Zerlegungsposition 202 versetzt werden (vgl. Fig. 1c).

Fig. 6d zeigt einen Ausschnitt des in Fig. 6b dargestellten ersten Schaftteils 220 mit dem Verriegelungsbereich 230 in der Entriegelungsstellung 234. Dargestellt ist der zweite Federarm 242, wobei die Darstellung und das in diesem Zusammenhang Beschriebene in gleicher Weise auf den ersten Federarm 241 übertragbar ist. Der Zylinder 268 befindet sich auf der Spitze der Wulst 246, d.h. zwischen der proximalen Flanke 264 und der distalen Flanke 266. Dadurch wird der zweite Federarm 242 entlang der vertikalen Richtung 404 ausgelenkt, wodurch der Schaft 200 von der Neutralposition 204 in die Zerlegungsposition 202 versetzt werden (vgl. Fig. 3b und 3c).

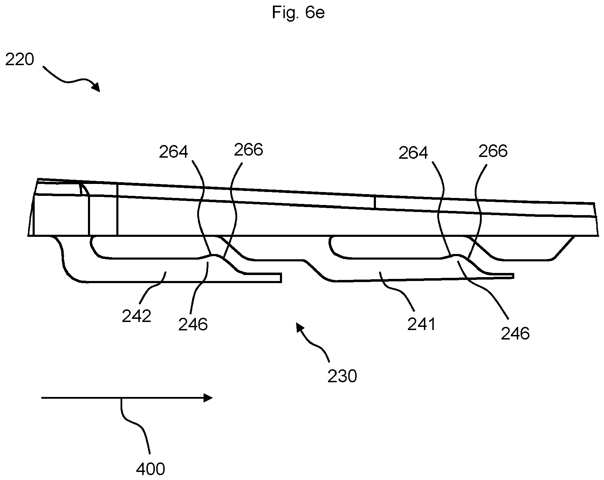

Fig. 6e zeigt eine Alternative zu dem in Fig. 6b dargestellten ersten Schaftteil 220. Die hier dargestellten beiden Wülste 246 sind voluminöser ausgeprägt und weisen gleichzeitig jeweils eine proximale Flanke 264 und eine distale Flanke 266 mit einer geringeren Steigung auf, als die in Fig. 6b dargestellten beiden Wülste 246 bzw. proximalen Flanken 264 und distalen Flanken 266. Mit der geringeren Steigung der proximalen Flanken 264 kann erreicht werden, dass dem versetzen des Verriegelungsbereiches 230 in die Entriegelungsposition 234 ein geringerer Widerstand entgegengesetzt wird, wodurch die Handhabbarkeit erleichtert wird. Mit dem vergrößerten Volumen der beiden Wülste 246 kann erreicht werden, dass deren Standzeit erhöht wird.

### Bezugszeichenliste

- 100: Knochenstanze
- 200: Schaft
- 202: Zerlegungsposition
- 204: Neutralposition
- 206: Stanzposition
- 220: erstes Schaftteil
- 221: erstes Führungselement
- 222: erstes Führungselement
- 223: erstes Führungselement
- 224: erstes Führungselement
- 225: erstes Führungselement
- 226: erstes Führungselement
- 228: Stanzelement
- 229: Führungsöffnung
- 230: Verriegelungsbereich
- 232: Neutralstellung
- 234: Entriegelungsstellung
- 240: Federbereich
- 241: erster Federarm
- 242: zweiter Federarm
- 244: Nase
- 246: Wulst
- 248: Freistich
- 250: zweites Schaftteil
- 251: zweites Führungselement
- 252: zweites Führungselement
- 253: zweites Führungselement
- 254: zweites Führungselement
- 258: Stanzaufnahme
- 260: Fixierbereich
- 262: Wulst
- 264: proximale Flanke
- 266: distale Flanke
- 268: Zylinder
- 270: erstes Anschlagselement
- 272: zweites Anschlagselement
- 274: Führungsöffnung
- 276: Aufnahme
- 280: Rückstellelement
- 282: Anschlag
- 284: Schaft
- 286: Feder
- 300: Griff
- 310: Verbindungsvorrichtung
- 312: Verbindungseinsatz
- 314: Anschlag
- 316: Gegenstück
- 318: Anschlag
- 319: Aufnahme
- 320: erstes Griffteil
- 322: erste Bewegung
- 324: zweite Bewegung
- 326: Vorsprung
- 330: erste Griffseite
- 332: zweite Griffseite
- 340: Griffkopf
- 341: Aufnahme
- 342: Aussparung
- 344: erster Lastübertragungsbereich
- 346: zweiter Lastübertragungsbereich
- 348: dritter Lastübertragungsbereich
- 350: zweites Griffteil
- 352: Steckelement
- 356: Vorsprung
- 360: erste Griffseite
- 362: zweite Griffseite
- 400: proximal-distale Richtung
- 402: horizontale Richtung
- 404: vertikale Richtung
- 410-410: Schnittansicht

## Patentansprüche

1. Zerlegbare Knochenstanze (100) aufweisend einen Schaft (200) und einen Griff (300), wobei
der Schaft (200) ein erstes Schaftteil (220) und ein zweites Schaftteil (250) aufweist, wobei das erste Schaftteil (220) zumindest ein erstes Führungselement (221) aufweist, und das zweite Schaftteil (250) zumindest ein zweites Führungselement (251) aufweist, wobei das erste Schaftteil (220) derart mit dem zweiten Schaftteil (250) verbunden ist, dass das erste Führungselement (221) in Eingriff mit dem zweiten Führungselement (251) ist und das erste Schaftteil (220) parallel zu einer sich von proximal nach distal ergebenden Richtung (400) relativ zum zweiten Schaftteil (250) bewegbar ist, wobei der Schaft (200) in eine Zerlegungsposition (202), in eine Neutralposition (204) und in eine Stanzposition (206) versetzbar ist, wobei
der Griff (300) ein erstes Griffteil (320) und ein zweites Griffteil (350) aufweist, wobei das erste Griffteil (320) bewegbar mit dem zweiten Schaftteil (250) verbunden ist, wobei das zweite Griffteil (350) mit dem zweiten Schaftteil (250) verbunden ist, wobei mittels einer ersten Bewegung (322) des ersten Griffteils (320), der Schaft (200) von der Neutralposition (204) in die Stanzposition (206) versetzbar ist,
wobei mittels einer zweiten Bewegung (324) des ersten Griffteils (320) der Schaft (200) von der Neutralposition (204) in die Zerlegungsposition (202) versetzbar ist, wobei
das erste Schaftteil (220) einen Verriegelungsbereich (230) aufweist, wobei der Verriegelungsbereich (230) von einer Neutralstellung (232) in eine Entriegelungsstellung (234) versetzbar ist, wobei der Verriegelungsbereich (230) derart ausgebildet ist, dass, wenn sich der Verriegelungsbereich (230) in der Neutralstellung (232) befindet, ein Versetzen des Schaftes (200) in die Zerlegungsposition (202) verhindert wird und **dadurch gekennzeichnet, dass** mittels der zweiten Bewegung (324) gleichzeitig der Verriegelungsbereich (230) in die Entriegelungsstellung (234) versetzt wird und der Schaft (200) in die Zerlegungsposition (202) versetzt wird.

2. Zerlegbare Knochenstanze (100) nach Anspruch 1, wobei der Verriegelungsbereich (230) einen auslenkbaren Federbereich (240) aufweist, wobei der Verriegelungsbereich (230) durch Auslenken des Federbereichs (240) in die Entriegelungsstellung (234) versetzbar ist.

3. Zerlegbare Knochenstanze (100) nach Anspruch 2, wobei der Federbereich (240) derart ausgebildet ist, dass dieser im Wesentlichen senkrecht zu der proximal-distalen Richtung (400) auslenkbar ist.

4. Zerlegbare Knochenstanze (100) nach Anspruch 3, wobei der Federbereich (240) im Wesentlichen parallel zu einer horizontalen Richtung (402) auslenkbar ist.

5. Zerlegbare Knochenstanze (100) nach Anspruch 3, wobei der Federbereich (240) im Wesentlichen parallel zu einer vertikalen Richtung (404) auslenkbar ist.

6. Zerlegbare Knochenstanze (100) nach einem der Ansprüche 2 bis 5, wobei der Federbereich (240) eine Aussparung (248) aufweist.

7. Zerlegbare Knochenstanze (100) nach einem der vorhergehenden Ansprüche, wobei das zweite Schaftteil (250) einen Fixierbereich (260) aufweist, wobei der Fixierbereich (260) derart ausgebildet und mit dem Verriegelungsbereich (230) interagierend angeordnet ist, dass, wenn sich der Verriegelungsbereich (230) in der Neutralstellung (232) befindet, durch den Fixierbereich (260) dem Versetzen des Schaftes (200) in die Zerlegungsposition (202) ein zusätzlicher Widerstand entgegenwirkt.

8. Zerlegbare Knochenstanze (100) nach Anspruch 7, wobei der Verriegelungsbereich (230) zumindest ein erstes Widerstandselement und/oder der Fixierbereich (260) zumindest ein zweites Widerstandselement aufweist, wobei das erste Widerstandselement und/oder das zweite Widerstandselement derart angeordnet und ausgebildet ist/sind, dass dem Versetzen des Verriegelungsbereiches (230) in die Entriegelungsstellung (234) ein zusätzlicher Widerstand entgegenwirkt.

9. Zerlegbare Knochenstanze (100) nach Anspruch 8 aufweisend ein erstes Widerstandselement und ein zweites Widerstandselement, wobei das erste Widerstandselement und das zweite Widerstandselement derart interagierend angeordnet und ausgebildet sind, dass dem Versetzen des Verriegelungsbereiches (230) in die Entriegelungsstellung (234) ein zusätzlicher Widerstand entgegenwirkt.

10. Zerlegbare Knochenstanze (100) nach Anspruch 9, insbesondere in Kombination mit Anspruch 4, wobei das erste Widerstandselement als Nase (244) ausgebildet ist und/oder das zweite Widerstandselement als Wulst (262) ausgebildet ist.

11. Zerlegbare Knochenstanze (100) nach Anspruch 9, insbesondere in Kombination mit Anspruch 5, wobei das erste Widerstandselement als Wulst (246) ausgebildet ist und/oder das zweite Widerstandselement als Zylinder (268) ausgebildet ist.

12. Zerlegbare Knochenstanze (100) nach einem der vorhergehenden Ansprüche, wobei die Knochenstanze (100) ferner ein insbesondere im zweiten Schaftteil (250) angeordnetes erstes Anschlagselement (270) aufweist, wobei das erste Anschlagselement (270) derart ausgebildet und ferner angeordnet ist, dass die Bewegbarkeit des ersten Griffteils (320) nach proximal hin begrenzt wird.

13. Zerlegbare Knochenstanze (100) nach einem der vorhergehenden Ansprüche, wobei die Knochenstanze (100) ferner ein insbesondere im zweiten Schaftteil (250) angeordnetes zweites Anschlagselement (272) aufweist, wobei das zweite Anschlagselement (272) derart ausgebildet und ferner angeordnet ist, dass die Bewegbarkeit des ersten Griffteils (320) nach distal hin begrenzt wird.

14. Zerlegbare Knochenstanze (100) nach einem der vorhergehenden Ansprüche, wobei die Knochenstanze (100) ferner ein, insbesondere im zweiten Schaftteil (250) angeordnetes, Rückstellelement (280) aufweist, wobei das Rückstellelement (280) derart ausgebildet und ferner angeordnet ist, dass nach Ausführen der ersten Bewegung (322) der Schaft (200) automatisch von der Stanzposition (206) wieder in die Neutralposition (204) versetzt wird und insbesondere der ersten Bewegung (322) ein, insbesondere zunehmender, Widerstand entgegenwirkt.

## Claims

1. Dismountable bone punch (100) having a shaft (200) and a handle (300), wherein
the shaft (200) has a first shaft part (220) and a second shaft part (250), wherein the first shaft part (220) has at least a first guide element (221), and wherein the second shaft part (250) has at least a second guide element (251), wherein the first shaft part (220) is connected to the second shaft part (250) such that the first guide element (221) is in engagement with the second guide element (251) and the first shaft part (220) is movable relative to the second shaft part (250) parallel to a direction (400) resulting from proximal to distal, wherein
the shaft (200) is displaceable into a dismounting position (202), a neutral position (204), and a punching position (206), wherein
the handle (300) has a first handle part (320) and a second handle part (350), wherein the first handle part (320) is movably connected to the second shaft part (250), wherein the second handle part (350) is connected to the second shaft part (250), wherein the shaft (200) is displaceable from the neutral position (204) into the punching position (206) by means of a first movement (322) of the first handle part (320),
wherein the shaft (200) is displaceable from the neutral position (204) into the dismounting position (202) by means of a second movement (324) of the first handle part (320), wherein
the first shaft part (220) has a locking region (230), wherein the locking region (230) is displaceable from a neutral position (232) into an unlocking position (234), wherein the locking region (230) is designed in such a way that, when the locking region (230) is in the neutral position (232), a displacement of the shaft (200) into the dismounting position (202) is prevented, and **characterized in that**, by means of the second movement (324), the locking region (230) is simultaneously displaced into the unlocking position (234) and the shaft (200) is displaced into the dismounting position (202).

2. Dismountable bone punch (100) according to claim 1, wherein the locking region (230) has a deflectable spring region (240), wherein the locking region (230) is displaceable into the unlocking position (234) by deflecting the spring region (240).

3. Dismountable bone punch (100) according to claim 2, wherein the spring region (240) is designed in such a way that it is deflectable substantially perpendicular to the proximal-distal direction (400).

4. Dismountable bone punch (100) according to claim 3, wherein the spring region (240) is deflectable substantially parallel to a horizontal direction (402).

5. Dismountable bone punch (100) according to claim 3, wherein the spring region (240) is deflectable substantially parallel to a vertical direction (404).

6. Dismountable bone punch (100) according to any one of claims 2 to 5, wherein the spring region (240) has a recess (248).

7. Dismountable bone punch (100) according to any one of the preceding claims, wherein the second shaft part (250) has a fixing region (260), wherein the fixing region (260) is designed and arranged in such a way that it is interacting with the locking region (230) such that, when the locking region (230) is in the neutral position (232), an additional resistance counteracts the displacement of the shaft (200) into the dismounting position (202) through the fixing region (260).

8. Dismountable bone punch (100) according to claim 7, wherein the locking region (230) has at least one first resistance element and/or the fixing region (260) has at least one second resistance element, wherein the first resistance element and/or the second resistance element is/are arranged and designed such that an additional resistance counteracts the displacement of the locking region (230) into the unlocking position (234).

9. Dismountable bone punch (100) according to claim 8, having a first resistance element and a second resistance element, wherein the first resistance element and the second resistance element are arranged and designed to be interacting in such a way that an additional resistance counteracts the displacement of the locking region (230) into the unlocking position (234).

10. Dismountable bone punch (100) according to claim 9, in particular in combination with claim 4, wherein the first resistance element is designed as a nose (244) and/or the second resistance element is designed as a bulge (262) .

11. Dismountable bone punch (100) according to claim 9, in particular in combination with claim 5, wherein the first resistance element is designed as a bulge (246) and/or the second resistance element is designed as a cylinder (268) .

12. Dismountable bone punch (100) according to any one of the preceding claims, wherein the bone punch (100) further has a first stop element (270) arranged in particular in the second shaft part (250), wherein the first stop element (270) is designed and further arranged in such a way that the movability of the first handle part (320) is limited in the proximal direction.

13. Dismountable bone punch (100) according to any one of the preceding claims, wherein the bone punch (100) further has a second stop element (272) arranged in particular in the second shaft part (250), wherein the second stop element (272) is designed and further arranged such that the movability of the first handle part (320) is limited in the distal direction.

14. Dismountable bone punch (100) according to any one of the preceding claims, wherein the bone punch (100) further has a restoring element (280), in particular arranged in the second shaft part (250), wherein the restoring element (280) is designed and further arranged in such a way that after performing the first movement (322), the shaft (200) is automatically displaced from the punching position (206) back into the neutral position (204) and an, in particular increasing, resistance counteracts in particular the first movement (322) .

## Revendications

1. Poinçon à os démontable (100) comprenant une tige (200) et une poignée (300), dans lequel
la tige (200) comprend une première partie de tige (220) et une seconde partie de tige (250), dans lequel la première partie de tige (220) comprend au moins un premier élément de guidage (221), et la seconde partie de tige (250) comprend au moins un second élément de guidage (251), dans lequel la première partie de tige (220) est reliée à la seconde partie de tige (250) de telle sorte que le premier élément de guidage (221) est en prise avec le second élément de guidage (251) et que la première partie de tige (220) peut être déplacée parallèlement à une direction (400) s'étendant de proximal à distal par rapport à la seconde partie de tige (250), dans lequel
la tige (200) peut être déplacée dans une position de démontage (202), dans une position neutre (204) et dans une position de poinçonnage (206), dans lequel
la poignée (300) comprend une première partie de poignée (320) et une seconde partie de poignée (350), dans lequel la première partie de poignée (320) est reliée de manière mobile à la seconde partie de tige (250), dans lequel la seconde partie de poignée (350) est reliée à la seconde partie de tige (250), dans lequel la tige (200) peut être déplacée de la position neutre (204) à la position de poinçonnage (206) au moyen d'un premier mouvement (322) de la première partie de poignée (320),
dans lequel la tige (200) peut être déplacée de la position neutre (204) à la position de démontage (202) au moyen d'un second mouvement (324) de la première partie de poignée (320), dans lequel
la première partie de tige (220) comprend une zone de verrouillage (230), dans lequel la zone de verrouillage (230) peut être déplacée d'une position neutre (232) dans une position de déverrouillage (234), dans lequel la zone de verrouillage (230) est conçue de telle sorte que, lorsque la zone de verrouillage (230) se trouve dans la position neutre (232), un déplacement de la tige (200) dans la position de démontage (202) est empêché et **caractérisé en ce qu'**au moyen du second mouvement (324), la zone de verrouillage (230) est déplacée simultanément dans la position de déverrouillage (234) et la tige (200) est déplacée dans la position de démontage (202).

2. Poinçon à os démontable (100) selon la revendication 1, dans lequel la zone de verrouillage (230) comprend une zone élastique (240) pouvant être déviée, dans lequel la zone de verrouillage (230) peut être amenée dans la position de déverrouillage (234) par déviation de la zone élastique (240).

3. Poinçon à os démontable (100) selon la revendication 2, dans lequel la zone élastique (240) est conçue de telle sorte qu'elle peut être déviée sensiblement perpendiculairement à la direction proximale-distale (400) .

4. Poinçon à os démontable (100) selon la revendication 3, dans lequel la zone élastique (240) peut être déviée sensiblement parallèlement à une direction horizontale (402) .

5. Poinçon à os démontable (100) selon la revendication 3, dans lequel la zone de ressort (240) peut être déviée sensiblement parallèlement à une direction verticale (404) .

6. Poinçon à os démontable (100) selon l'une des revendications 2 à 5, dans lequel la zone élastique (240) comprend un évidement (248).

7. Poinçon à os démontable (100) selon l'une des revendications précédentes, dans lequel la seconde partie de tige (250) comprend une zone de fixation (260), dans lequel la zone de fixation (260) est configurée et agencée pour interagir avec la zone de verrouillage (230) de telle sorte que, lorsque la zone de verrouillage (230) est dans la position neutre (232), la zone de fixation (260) oppose une résistance supplémentaire au déplacement de la tige (200) vers la position de démontage (202) .

8. Poinçon à os démontable (100) selon la revendication 7, dans lequel la zone de verrouillage (230) présente au moins un premier élément de résistance et/ou la zone de fixation (260) présente au moins un second élément de résistance, dans lequel le premier élément de résistance et/ou le second élément de résistance est/sont disposé(s) et conçu(s) de telle sorte qu'une résistance supplémentaire s'oppose au déplacement de la zone de verrouillage (230) dans la position de déverrouillage (234) .

9. Poinçon à os démontable (100) selon la revendication 8, comprenant un premier élément de résistance et un second élément de résistance, dans lequel le premier élément de résistance et le second élément de résistance étant disposés et conçus de manière à interagir de telle sorte qu'une résistance supplémentaire s'oppose au déplacement de la zone de verrouillage (230) dans la position de déverrouillage (234).

10. Poinçon à os démontable (100) selon la revendication 9, en particulier en combinaison avec la revendication 4, dans lequel le premier élément de résistance est réalisé sous la forme d'un nez (244) et/ou le second élément de résistance est réalisé sous la forme d'un bourrelet (262) .

11. Poinçon à os démontable (100) selon la revendication 9, en particulier en combinaison avec la revendication 5, dans lequel le premier élément de résistance est conçu comme un bourrelet (246) et/ou le second élément de résistance est conçu comme un cylindre (268).

12. Poinçon à os démontable (100) selon l'une des revendications précédentes, dans lequel le poinçon à os (100) comprend en outre un premier élément de butée (270) disposé en particulier dans la seconde partie de tige (250), dans lequel le premier élément de butée (270) est configuré et disposé en outre de manière à limiter la mobilité de la première partie de poignée (320) vers le côté proximal.

13. Poinçon à os démontable (100) selon l'une des revendications précédentes, dans lequel le poinçon à os (100) comprend en outre un second élément de butée (272) disposé en particulier dans la seconde partie de tige (250), dans lequel le second élément de butée (272) est configuré et disposé en outre de manière à limiter la mobilité de la première partie de poignée (320) vers le côté distal.

14. Poinçon à os démontable (100) selon l'une des revendications précédentes, dans lequel le poinçon à os (100) comprend en outre un élément de rappel (280) disposé en particulier dans la seconde partie de tige (250), dans lequel l'élément de rappel (280) est conçu et disposé en outre de telle sorte qu'après l'exécution du premier mouvement (322), la tige (200) est automatiquement déplacée à nouveau de la position de poinçonnage (206) dans la position neutre (204) et qu'en particulier une résistance, en particulier croissante, s'oppose au premier mouvement (322).
